Europäisches Patentamt
European Patent Office
Office européen des brevets

(19)

(11) Veröffentlichungsnummer: **0 239 543 A2**

# (12) EUROPÄISCHE PATENTANMELDUNG

(21) Anmeldenummer: **87810170.8**

(22) Anmeldetag: **23.03.87**

(51) Int. Cl.[4]: **C 07 K 9/00**
A 61 K 37/02

(30) Priorität: **27.03.86 CH 1227/86**

(43) Veröffentlichungstag der Anmeldung:
**30.09.87 Patentblatt 87/40**

(84) Benannte Vertragsstaaten:
**CH DE FR GB IT LI NL**

(71) Anmelder: **CIBA-GEIGY AG**
**Klybeckstrasse 141**
**CH-4002 Basel (CH)**

(72) Erfinder: **Baschang, Gerhard, Dr.**
**Bückenweg 7**
**CH-4126 Bettingen (CH)**

(54) **Neue Glucosederivate und Verfahren zu ihrer Herstellung.**

(57) Beschrieben sind 1,6-Anhydro-β-D-glucopyranose-derivate der Formel I,

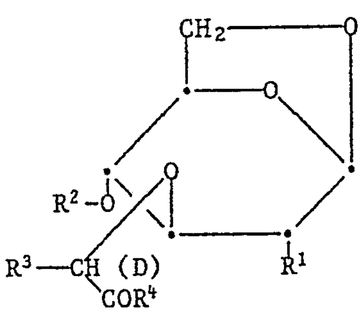

(I)

worin $R^1$ Azido oder Amino bedeutet, $R^2$ Wasserstoff oder eine Hydroxyschutzgruppe bedeutet, $R^3$ Wasserstoff oder Niederalkyl bedeutet und $R^4$ Hydroxy, Niederalkoxy oder einen Rest der Formel II bedeutet,

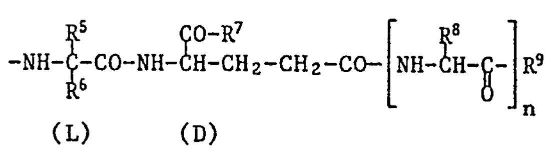

(II)

worin n für 0 oder 1 steht, $R^5$ Wasserstoff oder Niederalkyl bedeutet, $R^6$ Wasserstoff oder Niederalkyl, das unsubstituiert oder durch Hydroxy, Niederalkanoyloxy oder Phenyl substituiert ist, bedeutet, $R^7$ und $R^9$ unabhängig voneinander Hydroxy, Amino, Niederalkoxy, Arylniederalkoxy oder Niederalkanoyloxyniederalkoxy bedeuten und $R^8$ Wasserstoff oder unsubstituiertes oder durch Amino, Hydroxy, Niederalkanoylamino, Niederalkanoyloxy oder Guanidino substituiertes Niederalkyl bedeutet, sowie Stellungsisomere, worin die Positionen des Restes $R^2$ und der Gruppe -CH($R^3$)-CO-$R^4$ vertauscht sind, und Salze von solchen Verbindungen mit mindestens einer salzbildenden Gruppe.

Diese Verbindungen sind vielseitige Zwischenprodukte, z.B. zur Herstellung bekannter oder neuer Muramylpeptide. Sie gestatten die Einführung einer Vielfalt von Substituenten in 1- und 2-Stellung des Zuckerteils. Diejenigen Verbindungen der Formel I, worin $R^1$ Amino und $R^4$ einen Rest der Formel II bedeutet, sind ausserdem Immunstimulantien.

EP 0 239 543 A2

**Beschreibung**

## Neue Glucosederivate und Verfahren zu ihrer Herstellung

Die Erfindung betrifft 1,6-Anhydro-β-D-glucopyranose-derivate und Verfahren zu ihrer Herstellung. Die genannten Anhydro-D-glucosederivate können unter anderem als vielseitige Zwischenprodukte, z.B. zur Herstellung bekannter oder neuer Muramylpeptide, verwendet werden.

Die bisher bekannten Muramylpeptide weisen in der Regel in 2-Stellung des Zuckerteils eine acylierte, d.h. alkanoylierte oder benzoylierte Aminogruppe auf. Was die glykosidierten Muramylpeptide angeht, so weisen diese in der Regel in 1-Stellung einen Benzyl-, Methyl- oder Ethylsubstituenten auf. Diese beschränkte Variation der Substituenten in 1- und 2-Stellung des Muramylpeptidmoleküls steht im grossen Gegensatz zu der breiten Substituentenvariation an anderen Positionen des Muramylpeptidmoleküls. Die Ursache für diesen Gegensatz liegt in der schwierigen Herstellbarkeit von Muramylpeptiden, die andere als die genannten Substituenten in 1- und 2-Stellung aufweisen. Ebenfalls aufgrund ihrer schwierigen Herstellbarkeit sind zwar zahlreiche in 6-Stellung substituierte und in 4-Stellung unsubstituierte sowie 4,6-disubstituierte Muramylpeptide bekannt, aber synthetisch hergestellte, in 4-Stellung substituierte und in 6-Stellung unsubstituierte Muramylpeptide sind praktisch unbekannt, obwohl derartige Muramylpeptide einen wichtigen Bestandteil der in der Bakterienzellwand enthaltenen hochmolekularen, polymeren Peptidoglykane bilden.

Bei der Muraminsäuresynthese aus Glucosamin und einem Milchsäurederivat müssen die freien funktionellen Gruppen des Glucosamins mit Ausnahme der an der Reaktion teilnehmenden 3-Hydroxy-Gruppe geschützt werden. Zum Schutz der 1-Hydroxy-Gruppe wird fast immer die Benzylgruppe verwendet, die nach erfolgter Reaktion durch eine langwierige, manchmal tagelange Hydrierung wieder abgespalten wird.

Der vorliegenden Erfindung liegt die Aufgabe zugrunde, einen neuen und einfachen Zugang, bei welchem die 1-Hydroxy-Gruppe nicht mittels Benzyl geschützt ist, zu solchen Muramylpeptiden zu öffnen und entsprechend substituierte Muramylpeptide herzustellen.

Die Erfindung betrifft insbesondere 1,6-Anhydro-β-D-glucopyranosederivate der Formel I,

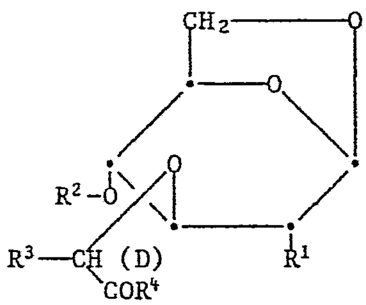

(I)

worin $R^1$ Azido oder Amino bedeutet, $R^2$ Wasserstoff oder eine Hydroxyschutzgruppe bedeutet, $R^3$ Wasserstoff oder Niederalkyl bedeutet und $R^4$ Hydroxy, Niederalkoxy oder einen Rest der Formel II bedeutet,

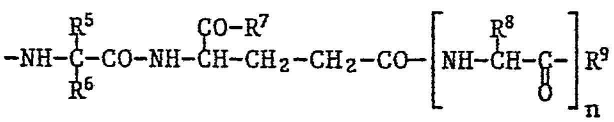

(L) (D)

(II)

worin n für 0 oder 1 steht, $R^5$ Wasserstoff oder Niederalkyl bedeutet, $R^6$ Wasserstoff oder Niederalkyl, das unsubstituiert oder durch Hydroxy, Niederalkanoyloxy oder Phenyl substituiert ist, bedeutet, $R^7$ und $R^9$ unabhängig voneinander Hydroxy, Amino, Niederalkoxy, Arylniederalkoxy oder Niederalkanoyloxyniederalkoxy bedeuten und $R^8$ Wasserstoff oder unsubstituiertes oder durch Amino, Hydroxy, Niederalkanoylamino, Niederalkanoyloxy oder Guanidino substituiertes Niederalkyl bedeutet, sowie Stellungsisomere, worin die Positionen des Restes $R^2$ und der Gruppe -CH($R^3$)-CO-$R^4$ vertauscht sind, und Salze von solchen Verbindungen mit mindestens einer salzbildenden Gruppe.

Im Falle asymmetrischer Substitution ist die Konfiguration an den Atomen C-$R^3$, C-$R^6$ beziehungsweise C-CO-$R^7$ (D), (L) bzw, wie in den Formeln I und II angegeben. Die Konfiguration am C-$R^8$ ist im Falle asymmetrischer Substitution (L) oder (D), vorzugsweise (L).

Als Rest $R^2$ geeignete Hydroxyschutzgruppen im besonderen und Schutzgruppen im allgemeinen sowie deren Einführung und Abspaltung sind beispielsweise beschrieben in "Protective Groups in Organic Chemistry", Plenum Press, London, New York 1973, und in "Methoden der oganischen Chemie", Houben-Weyl, 4. Auflage, Bd. 15/1, Georg-Thieme-Verlag, Stuttgart 1974 sowie in Theodora W. Greene, "Protective Groups in Organic Synthesis, John Wiley & Sons, New York 1981. Charakteristisch für

Schutzgruppen ist, dass sie leicht d.h. ohne dass unerwünschte Nebenreaktionen stattfinden, z.B. solvolytisch, reduktiv, photolytisch oder auch unter physiologischen Bedingungen abspaltbar sind.

Hydroxyschutzgruppen sind z.B. Acylreste, wie gegebenenfalls, z.B. durch Halogen, substituiertes Niederalkanoyl, wie 2,2-Dichloracetyl, oder Acylreste von Kohlensäurehalbestern, insbesondere tert.-Butyloxycarbonyl, gegebenenfalls substituiertes Benzyloxycarbonyl, z.B. 4-Nitro-benzyloxycarbonyl, oder Diphenylmethoxycarbonyl, oder 2-Halogen-niederalkoxycarbonyl, wie 2,2,2-Trichlorethoxycarbonyl, ferner Trityl oder Formyl, oder organische Silyl- oder Stannylreste, ferner leicht abspaltbare verethernde Gruppen, wie tert.-Niederalkyl, z.B. tert.-Butyl, 2-oxa- oder 2-thia-aliphatische oder cycloaliphatische Kohlenwasserstoffreste, in erster Linie 1-Niederalkoxyniederalkyl oder 1-Niederalkylthio-niederalkyl, z.B. Methoxymethyl, 1-Methoxy-ethyl, 1-Ethoxy-ethyl, Methylthiomethyl, 1-Methyl thioethyl oder 1-Ethylthioethyl, oder 2-Oxa- oder 2-Thiacycloalkyl mit 5-6 Ringatomen, z.B. Tetrahydrofuryl oder 2-Tetrahydropyranyl oder entsprechende Thiaanaloge, sowie gegebenenfalls substituiertes 1-Phenylniederalkyl, wie gegebenenfalls substituiertes Benzyl oder Diphenylmethyl, wobei als Substituenten der Phenylreste z.B. Halogen, wie Chlor, Niederalkoxy, wie Methoxy und/oder Nitro in Frage kommen.

Die oben und im folgenden erwähnten organischen Silyl- oder Stannylreste enthalten vorzugsweise Niederalkyl, insbesondere Methyl, als Substituenten der Silizium- oder Zinn-Atome. Entsprechende Silyl-oder Stannylgruppen sind in erster Linie Triniederalkylsilyl, insbesondere Trimethylsilyl, ferner Dimethyl-tert.-butylsilyl oder durch Phenyl, welches unsubstituiert oder substituiert sein kann, und/oder Niederalkyl trisubstituiertes Silyl, insbesondere Tert.-butyl-diphenyl-silyl, oder entsprechend substituiertes Stannyl, z.B. Tri-n-butylstannyl.

Zur Herstellung von Verbindungen der Formel I über Zwischenprodukte der untengezeigten Formel V verwendet man vorzugsweise Hydroxyschutzgruppen, die nicht vom 4-O-Atom zum 3-O-Atom des Pyranoseteils wandern können, d.h. Schutzgruppen, die über ein gesättigtes C-Atom, von dem 4 Einfachbindungen ausgehen, gebunden sind, z.B. Benzyl, Tetrahydropyranyl, Allyl oder vorzugsweise Trityl. Eine Hydroxyschutzgruppe $R^2$ ist daher vorzugsweise eine solche nicht wandernde Hydroxyschutzgruppe.

Niederalkyl $R^3$ oder $R^5$ ist vorzugsweise $C_{1-3}$-Alkyl, insbesondere Methyl.

Niederalkoxy $R^4$ ist insbesondere $C_{1-4}$-Alkoxy, z.B. tert.Butoxy oder vorzugsweise Methoxy.

Unsubstituiertes Niederalkyl $R^6$ oder $R^8$ ist vorzugsweise $C_{1-4}$-Alkyl, z.B. Ethyl, Isopropyl, 2-Methylpropyl, sek.Butyl oder insbesondere Methyl.

Durch Phenyl, Hydroxy oder Niederalkanoyloxy substituiertes Niederalkyl $R^6$ ist vorzugsweise entsprechend substituiertes $C_{1-2}$-Alkyl, z.B. Benzyl, Hydroxymethyl, 1-Hydroxy-ethyl, Acetoxy-methyl oder 1-Acetoxy-ethyl.

Niederalkoxy $R^7$ oder $R^9$ ist vorzugsweise $C_{1-4}$-Alkoxy, z.B. Methoxy, n-Butyloxy oder tert. Butyloxy.

Aryl als Teil von Arylniederalkoxy $R^7$ oder $R^9$ ist insbesondere unsubstituiertes oder durch einen oder mehrere, z.B. 1-3, der untengenannten Arylsubstituenten substituiertes Phenyl oder Naphthyl, vorzugsweise unsubstituiertes Phenyl.

Arylsubstituenten sind insbesondere Niederalkyl, z.B. Methyl, Phenyl, Halogen, Hydroxy, Niederalkoxy, z.B. Methoxy, Niederalkanoyloxy, z.B. Acetoxy, Amino, Mono- oder Diniederalkylamino, z.B. Mono- oder Dimethylamino oder Niederalkanoylamino, z.B. Acetylamino.

Arylniederalkoxy $R^7$ und/oder $R^9$ ist durch einen oder mehrere, z.B. 1-3, vorzugsweise 1 oder 2, Arylreste substituiertes Niederalkoxy, z.B. Arylmethoxy, wie insbesondere Benzyloxy oder Benzhydryloxy.

Niederalkanoyloxyniederalkoxy $R^7$ oder $R^9$ ist vorzugsweise Niederalkanoyloxymethoxy, z.B. Pivaloyloxymethoxy.

Durch Amino oder Niederalkanoylamino substituiertes Niederalkyl $R^8$ ist vorzugsweise 4-Amino-n-butyl oder 4-Niederalkanoylamino-n-butyl.

Durch Hydroxy oder Niederalkanoyloxy substituiertes Niederalkyl $R^{11}$ ist insbesondere entsprechend substituiertes $C_{1-2}$-Alkyl, z.B. Hydroxymethyl, 1-Hydroxy-ethyl, Niederalkanoyloxymethyl oder 1-Niederalkanoyloxy-ethyl. Durch Guanidino substituiertes Niederalkyl $R^{11}$ ist vorzugsweise 3-Guanidino-n-propyl.

Niederalkanoylamino ist z.B. Acetamino. Niederalkanoyloxy ist z.B. Acetoxy.

Die obengenannten Stellungsisomeren sind Verbindungen der Formel III,

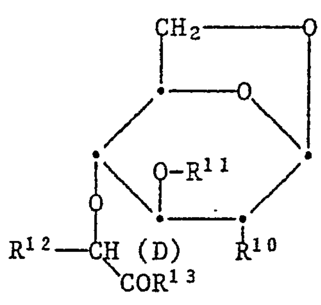

(III)

worin $R^{10}$ Azido oder Amino bedeutet, $R^{11}$ Wasserstoff oder eine Hydroxyschutzgruppe bedeutet, $R^{12}$

Wasserstoff oder Niederalkyl bedeutet und $R^{13}$ Hydroxy, Niederalkoxy oder einen Rest der Formel IV bedeutet.

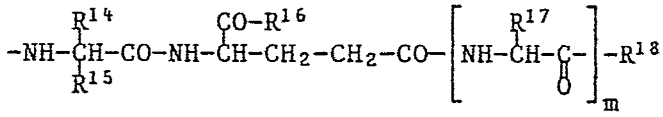 (IV)

(L) (D)

worin m für 0 oder 1 steht, $R^{14}$ Wasserstoff oder Niederalkyl bedeutet, $R^{15}$ Wasserstoff oder Niederalkyl, das unsubstituiert oder durch Hydroxy, Niederalkanoyloxy oder Phenyl substituiert ist, bedeutet, $R^{16}$ und $R^{18}$ unabhängig voneinander Hydroxy, Amino, Niederalkoxy, Arylniederalkoxy oder Niederalkanoyloxyniederalkoxy bedeuten und $R^{17}$ Wasserstoff oder unsubstituiertes oder durch Amino, Hydroxy, Niederalkanoylamino, Niederalkanoyloxy oder Guanidino substituiertes Niederalkyl bedeutet, und Salze von solchen Verbindungen mit mindestens einer salzbildenden Gruppe.

Die zur Definition der Substituenten in den Formeln III und IV verwendeten Begriffe sind so zu verstehen, wie oben im Zusammenhang mit den Formeln I und II angegeben ist.

Bevorzugterweise betrifft die Erfindung die Erfindungen der Formel I.

Die vor- und nachstehend verwendeten Allgemeinbegriffe haben vorzugsweise folgende Bedeutungen:

Das Präfix "Nieder" bezeichnet Reste bis und mit 7, insbesondere bis und mit 4, Kohlenstoffatomen.

Halogen ist insbesondere Chlor oder Brom, ferner Fluor oder Iod.

Salzbildende Gruppen in einer Verbindung der Formel I sind saure Gruppen, z.B. freie Carboxylgruppen, oder basische Gruppen, wie insbesondere freie Amino- oder Guanidinogruppen. Verbindungen der Formel I, die eine Trimethylammoniogruppe aufweisen, liegen in Salzform vor. Je nach der Art der salzbildenden Gruppe bilden die Verbindungen der Formel I Metall- oder Ammoniumsalze oder Säureadditionssalze. Salze einer Verbindung der Formel I, worin $R^1$ Amino bedeutet, sind vorzugsweise pharmazeutisch verwendbar und nicht toxisch, z.B. Alkalimetall- oder Erdalkalimetallsalze, z.B. Natrium-, Kalium-, Magnesium- oder Calciumsalze, oder Salze mit Ammoniak oder geeigneten organischen Aminen, wobei in erster Linie aliphatische, cycloaliphatische, cycloaliphatisch-aliphatische oder araliphatische primäre, sekundäre oder tertiäre Mono-, Di- oder Polyamine, sowie heterocyclische Basen für die Salzbildung in Frage kommen, wie Niederalkylamine, z.B. Triethylamin, Hydroxyniederalkylamine, z.B. 2-Hydroxy-ethylamin, Bis-(2-hydroxyethyl)-amin, 2-Hydroxy-ethyl-diethyl-amin oder Tri-(2-hydroxyethyl)-amin, basische aliphatische Ester von Carbonsäuren, z.B. 4-Amino-benzoesäure-2-diethylaminoethylester, Niederalkylenamine, z.B. 1-Ethylpiperidin, Cycloalkylamine, z.B. Dicyclohexylamin, oder Benzylamine, z.B. N, N'-Dibenzylethylendiamin, ferner Basen vom Pyridintyp, z.B. Pyridin, Collidin oder Chinolin. Verbindungen der Formel I mit mindestens einer basischen Gruppe können Säureadditionssalze, z.B. mit anorganischen Säuren, wie Salzsäure, Schwefelsäure oder Phosphorsäure, oder mit geeigneten organischen Carbon- oder Sulfon säuren, z.B. Trifluoressigsäure, sowie mit Aminosäuren, wie Arginin und Lysin, bilden. Bei Anwesenheit von mehreren sauren oder basischen Gruppen können Mono-oder Polysalze gebildet werden. Verbindungen der Formel I mit einer sauren, z.B. freien Carboxylgruppe, und einer freien basischen, z.B. einer Aminogruppe, können auch in Form von inneren Salzen, d.h. in zwitterionischer Form vorliegen, oder es kann ein Teil des Moleküls als inneres Salz, und ein anderer Teil als normales Salz vorliegen.

Zur Isolierung oder Reinigung sowie bei den als Zwischenprodukte weiterverwendeten Verbindungen können auch pharmazeutisch ungeeignete Salze Verwendung finden. Zur therapeutischen Anwendung gelangen jedoch nur die pharmazeutisch verwendbaren, nicht-toxischen Salze, die deshalb bevorzugt werden.

Die Verbindungen der Formeln I und III sind vielseitig verwendbare Zwischenprodukte. Die Verbindungen der Formel I können in bekannte Muramylpeptide überführt werden, die ihrerseits als Adjuvantien in der Immunologie, als Antitumormittel oder zur Prophylaxe und Therapie von Virusinfektionen eingesetzt werden können. Die Verbindungen der Formel III können in stellungsisomere Muramylpeptide mit dem gleichen Anwendungsbereich überführt werden. Die Verbindungen der Formeln I und III, worin $R^1$ Azido ist, können durch Reduktion mit einem geeigneten Reduktionsmittel, z.B. mit Schwefelwassestoff in Pyridin, mit Triphenylphosphin in Tetrahydrofuran-Wasser oder durch Hydrierung an einem geeigneten Hydrierkatalysator, z.B. Palladium auf einen geeigneten Träger, in Verbindungen der Formeln I bzw. III überführt werden, worin $R^1$ Amino bedeutet. Freies Amino $R^1$ kann in an sich bekannter Weise acyliert werden.

Eine Derivatisierung in 4-Stellung sowie insbesondere in 1-Stellung des Zuckerteils wird vorteilhafterweise mit denjenigen Verbindungen der Formeln I und III durchgeführt, worin $R^1$ Azido ist. Azido $R^1$ kann auch in an sich bekannter Weise in andere Reste als Amino überführt werden. Zur Ueberführung einer Verbindung der Formel I, worin $R^1$ Amino und $R^4$ einen Rest der Formel II bedeuten, in bekannte Muramylpeptide acyliert man zunächst die Aminogruppe in an sich bekannter Weise, öffnet dann den Anhydroring mit Wasser in einem geeigneten organischen Lösungsmittel, wie Dimethoxyethan, oder mit einem Alkohol, jeweils in Gegenwart katalytischer Mengen einer starken Säure, z.B. Trifluormethansulfonsäure, und setzt, falls erforderlich, die Hydroxygruppe in 4-Stellung in Freiheit. Analog überführt man die Verbindungen der Formel III, worin $R^{10}$ Amino und $R^{13}$ einen Rest der Formel IV bedeutet, in die stellungsisomeren Muramylpeptide.

In Verbindungen der Formeln I und III kann der 1,6-Anhydro-Ring z.B. auch durch Umsetzung mit einem reaktionsfähigen Carbonsäurederivat, erforderlichenfalls in Gegenwart von katalytischen Mengen einer starken Säure, geöffnet werden, wobei die 1,6-O-Diacyl-Derivate oder, wenn die 4-Hydroxy-Gruppe frei ist oder im Verlauf der Reaktion intermediär freigesetzt wird, die 1,4,6-O-Triacyl-Derivate entstehen. Beispielsweise kann man als reaktionsfähiges Carbonsäurederivat ein Anhydrid, z.B. Acetanhydrid, und als starke Säure Trifluormethansulfonsäure verwenden.

Die Verbindungen der Formel I, worin $R^1$ Amino, $R^2$ Wasserstoff, $R^3$ Wasserstoff der Niederalkyl und $R^4$ einen Rest der Formel II bedeuten, sind aber nicht nur Zwischenprodukte, sondern selbst pharmakologisch wirksam. Insbesondere weisen sie selbst eine immunstimulierende, z.B. Makrophagen aktivierende Wirkung auf. So vermögen diese Verbindungen der Formel I, z.B. inkorporiert in multilamellären Liposomen oder in phosphatgepufferter physiologischer Kochsalzlösung (PBS), Makrophagen derart zu aktivieren, dass diese körpereigenen Abwehrzellen in der Lage sind, Tumorzellen abzutöten (Zytotoxizität) oder an ihrem Wachstum zu hindern (Zytostase). Die Induktion tumorizider und tumoristatischer Alveolarmakrophagen der Ratte in vitro und in situ lässt sich z.B. mit folgendem Versuch zeigen:

Alveolarmakrophagen werden durch Lungenspülung mit Kulturmedium erhalten. Diese Makrophagen werden entweder durch Injektion der Testsubstanzen in der Ratten (intravenös oder intranasal, in situ-Aktivierung) oder durch eine 24stündige Vorinkubation mit einer Verbindung der Formel I im $CO_2$-Inkubator (in vitro-Aktivierung) aktiviert. Die so aktivierten Makrophagen werden nun für weitere 72 Stunden mit Tumorzellen inkubiert.

Um tumorizide Aktivitäten der Makrophagen zu messen, werden die Tumorzellen vor der 72 Stunden-Inkubation mit $^{125}$I-Ioddeoxyuridin markiert. Die nicht abgetöteten Tumorzellen können nach Wegwaschen der durch lysierte Tumorzellen freigewordenen Radioaktivität anhand der verbleibenden Radioaktivität gemessen werden.

Um tumoristatische Aktivitäten der Makrophagen zu erfassen, wird den Kulturen 8 Stunden vor dem Ende der 72 Stunden-Inkubation $^3$H-Thymidin zugesetzt und danach die $^3$H-Thymidininkorporation in die Tumorzellen gemessen. In vitro können die Substanzen sowohl gelöst in PBS als auch inkorporiert in Liposomen bereits in Dosen von 20 Nanogramm/0,2 ml Kultur tumorizide Ratten-Alveolarmakrophagen induzieren. Bei Ratten bewirkt eine einmalige intravenöse Applikation der Verbindungen inkorporiert in Liposomen bei einer Dosis von 160 µg/Tier eine Induktion von tumoriziden und tumoristatischen Alveolarmakrophagen. Darüberhinaus bewirkt eine einmalige intranasale Applikation der Substanzen in PBS bei einer Dosis von 25 µg/Ratte die Induktion von tumoriziden Alveolarmakrophagen.

Die Verbindungen der Formel I können somit bei Warmblütern einschliesslich des Menschen auch zur Therapie von Tumorerkrankungen verwendet werden, insbesondere z.B. zur Vermeidung der Bildung von Metastasen, z.B. bei operativer Entfernung des Primärtumors.

Die therapeutische Dosis für Warmblüter von etwa 70 kg Körpergewicht liegt zwischen 0,1 mg und 25 mg, vorzugsweise zwischen 0,1 und 1 mg, z.B. bei 0,5 mg, insbesondere bei oraler Applikation. Die Dosierung bei topischer, insbesondere intranasaler Applikation, liegt bis zum Faktor 10 niedriger. Bei Bedarf kann man die Verabreichung der Verbindungen der Formel I bis zum Eintritt einer Besserung der Erkrankung wiederholen. Oft genügt jedoch eine einmalige Applikation.

Falls 1,6-Anhydro-β-D-muraminsäure, das als Bestandteil des sogenannten "Schlaffaktors" in der Natur vorkommt, zum Stand der Technik gehört, betrifft die Erfindung diese Verbindung nicht als solche.

Alle Verbindungen der Formel I können als Zwischenprodukte zur Herstellung bekannter oder neuer Muramylpeptide verwendet werden.

Als Zwischenprodukte dienen insbesondere diejenigen Verbindungen der Formel I, worin $R^1$ Azido bedeutet, und/oder $R^2$ eine vorzugsweise über ein gesättigtes C-Atom gebundene Hydroxyschutzgruppe, z.B. Allyl, Benzyl, Tetrahydropyranyl oder insbesondere Trityl, bedeutet, und/oder $R^4$ Niederalkoxy oder vorzugsweise Hydroxy bedeutet, oder ein Salz einer solchen Verbindung mit mindestens einer salzbildenden Gruppe.

Bevorzugte Zwischenprodukte sind die Verbindungen der Formel I, worin $R^1$ Azido oder Amino, $R^2$ Wasserstoff oder eine Hydroxyschutzgruppe, $R^3$ Wasserstoff oder Niederalkyl und $R^4$ Hydroxy oder Niederalkoxy bedeuten, insbesondere solche, worin $R^1$ Azido, $R^2$ eine Hydroxyschutzgruppe, $R^3$ Wasserstoff oder Methyl und $R^4$ Hydroxy bedeuten, und Salze von solchen Verbindungen mit mindestens einer salzbildenden Gruppe.

Ein Teil der Verbindungen der Formel I kann nicht nur als Zwischenprodukt, sondern auch als pharmakologisch wirksames Endprodukt verwendet werden. Pharmakologisch wirksam sind dienigen Verbindungen der Formel I, worin $R^1$ Amino, $R^2$ Wasserstoff oder eine pharmazeutisch verwendbare Hydroxyschutzgruppe, z.B. Niederalkanoyl, $R^3$ Wasserstoff oder Niederalkyl und $R^4$ einen Rest der wie oben definierten Formel II bedeuten, insbesondere einen solchen Rest der Formel II, worin n für 0 oder 1 steht, $R^5$ Wasserstoff oder $C_{1-3}$-Alkyl bedeutet, $R^6$ Wasserstoff, $C_{1-4}$-Alkyl oder durch Hydroxy, Niederalkanoyloxy oder Phenyl substituiertes $C_{1-2}$-Alkyl bedeutet, $R^7$ und $R^9$ unabhängig voneinander Hydroxy, Amino, Niederalkoxy, Niederalkanoyloxymethoxy, oder durch 1-3 Phenylreste substituiertes Methoxy bedeuten und $R^8$ Wasserstoff, $C_{1-4}$-Alkyl, 4-Amino-n-butyl, 4-Niederalkanoylamino-n-butyl, 3-Guanidino-n-propyl oder durch Hydroxy oder Niederalkanoyloxy substituiertes $C_{1-2}$-Alkyl bedeutet, und pharmazeutisch verwendbare Salze von solchen Verbindungen mit mindestens einer salzbildenden Gruppe.

Bevorzugte Endprodukte sind die Verbindungen der Formel I, worin $R^1$ Amino, $R^2$ Wasserstoff, $R^3$

Wasserstoff oder Niederalkyl und $R^4$ einen Rest der wie oben definierten Formel II bedeuten, und pharmazeutisch verwendbare Salze von solchen Verbindungen mit mindestens einer salzbildenden Gruppe.

Besonders bevorzugte Endprodukte sind die obengenannten Verbindungen der Formel I, worin $R^4$ einen Rest der Formel II bedeutet, worin n für 0 steht, $R^5$ Wasserstoff bedeutet, $R^6$ $C_{1-4}$-Alkyl bedeutet und $R^7$ und $R^9$ unabhängig voneinander Hydroxy, Amino, $C_{1-4}$-Alkoxy oder Benzyloxy bedeuten, und pharmazeutisch verwendbare Salze von solchen Verbindungen mit mindestens einer salzbildenden Gruppe.

Hervorzuheben sind auch diejenigen Verbindungen der Formel I, worin $R^1$ Azido, $R^2$ Wasserstoff oder eine über ein gesättigtes C-Atom gebundene Hydroxyschutzgruppe, $R^3$ Wasserstoff oder Methyl und $R^4$ Hydroxy bedeuten, und ihre Salze, sowie diejenigen Verbindungen der Formel I, worin $R^1$ Azido oder Amino, $R^2$ Wasserstoff oder eine über ein gesättigtes C-Atom gebundene Hydroxyschutzgruppe, $R^3$ Wasserstoff und $R^4$ Hydroxy bedeuten, und ihre Salze.

Am meisten bevorzugt sind die in den Beispielen genannten Verbindungen der Formeln I oder III.

Die Verbindungen der Formel I oder Salze von solchen Verbindungen mit mindestens einer salzbildenden Gruppe werden z.B. hergestellt, indem man

a) zur Herstellung einer Verbindung der Formel I, worin $R^1$ Azido und $R^4$ Hydroxy oder Niederalkoxy bedeuten, eine Verbindung der Formel VI,

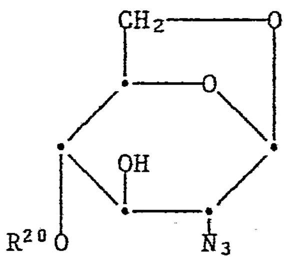

(VI)

worin $R^{20}$ eine Hydroxyschutzgruppe bedeutet, oder ein reaktionsfähiges Derivat davon mit einer Verbindung der Formel VII,

$$X - \overset{R^3}{CH} - COR^{19} \qquad (VII)$$

worin X eine reaktionsfähige veresterte Hydroxygruppe bedeutet, $R^{19}$ Hydroxy oder Niederalkoxy bedeutet und $R^3$ eine der obengenannten Bedeutungen hat, umsetzt und, wenn erwünscht, zur Herstellung von Verbindungen der Formel I, worin $R^2$ Wasserstoff bedeutet, die Hydroxyschutzgruppe $R^{20}$ abspaltet, oder

b) zur Herstellung einer Verbindung der Formel I, worin $R^1$ Azido und $R^4$ einen Rest der Formel II bedeuten, eine Verbindung der Formel VIII,

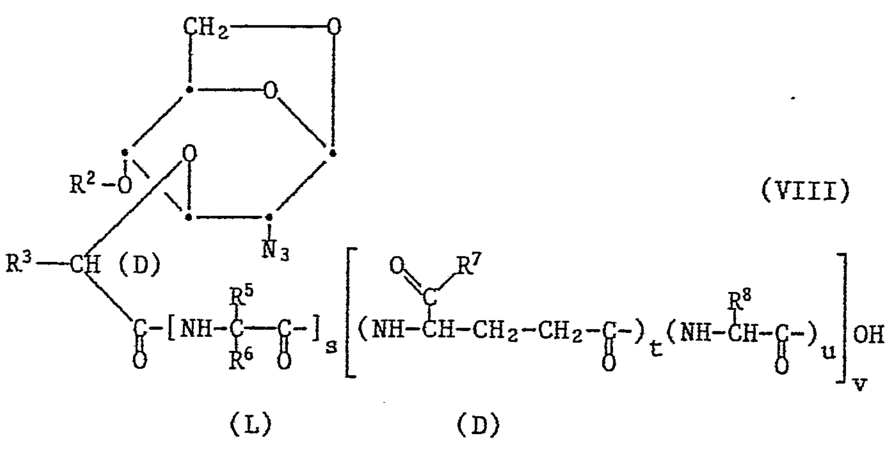

(VIII)

worin s, t, u und v unabhängig voneinander für 0 oder 1 stehen und worin die Substituenten die obengenannten Bedeutungen haben, wobei in einer Verbindung der Formel VIII vorhandene freie funktionelle Gruppen mit Ausnahme der Gruppe, die an der Reaktion teilnehmen soll, erforderlichenfalls durch leicht abspaltbare Schutzgruppen geschützt sind, oder ein reaktionsfähiges Carbonsäurederivat davon mit einer Verbindung der Formel IX,

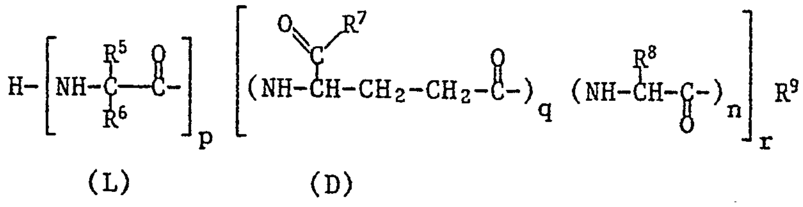

(IX)

worin p, q und r unabhängig voneinander für 0 oder 1 stehen und die übrigen Symbole und Substituenten die obengenannten Bedeutungen haben, wobei in einer Verbindung der Formel IX vorhandene freie funktionelle Gruppen mit Ausnahme der Gruppe, die an der Reaktion teilnehmen soll, erforderlichenfalls durch leicht abspaltbare Schutzgruppen geschützt sind, wobei p, q und r für 1 stehen, wenn im Reaktionspartner der Formel VIII s und v für 0 stehen, oder p für 0 und q und r für 1 stehen, wenn s für 1 und v für 0 stehen, oder p und q für 0 und r für 1 stehen, wenn s, t und v für 1 und u für 0 stehen oder zur Herstellung von Verbindungen der Formel I, worin n für 1 steht, p und r für 0 stehen, wenn s, t, u und v für 1 stehen, oder mit einem reaktionsfähigen Derivat davon umsetzt und vorhandene Schutzgruppen erforderlichenfalls abspaltet, oder

c) zur Herstellung einer Verbindung der Formel I, worin $R^1$ Amino bedeutet, eine Verbindung der Formel I, worin $R^1$ Azido bedeutet und die übrigen Substituenten die obengenannten Bedeutungen haben, wobei vorhandene freie funktionelle Gruppen mit Ausnahme der Gruppe, die an der Reaktion teilnehmen soll, erforderlichenfalls durch leicht abspaltbare Schutzgruppen geschützt sind, mit einem geeigneten Reduktionsmittel in die Aminoverbindung überführt und vorhandene Schutzgruppen erforderlichenfalls abspaltet,

und, wenn erwünscht, nach Durchführung eines der Verfahren a-c) eine erhaltene Verbindung der Formel I mit mindestens einer salzbildenden Gruppe in ihr Salz überführt oder ein erhaltenes Salz einer Verbindung der Formel I in die freie Verbindung umwandelt, und/oder ein erhaltenes Isomerengemisch auftrennt, und/oder zwecks Ueberführung einer erhaltenen Verbindung der Formel I in eine andere Verbindung der Formel I freies Hydroxy im Rest $R^6$ und/oder $R^8$ verestert oder freies Amino im Rest $R^8$ acyliert oder freies Carboxy $-COR^7$ und/oder $-COR^9$ verestert oder amidiert.

Die Durchführung der obengenannten Verfahrensvarianten wird im folgenden näher erläutert:

Verfahren a)

Ein reaktionsfähiges Derivat einer Verbindung der Formel VI ist z.B. eine geeignete Metalloxyverbindung, wie sie z.B. durch Umsetzung einer Verbindung der Formel VI mit einer geeigneten Base, wie einem Alkalimetallhydrid oder -amid, z.B. mit Natriumhydrid, erhalten werden kann.

Als Hydroxyschutzgruppen $R^{20}$ können die oben für $R^2$ beschriebenen verwendet werden. Vorzugsweise verwendet man solche Hydroxyschutzgruppen, die nicht vom 4-0-Atom zum 3-0-Atom wandern können, d.h. Schutzgruppen, die über ein gesättigtes C-Atom gebunden sind, z.B. Trityl.

Reaktionsfähiges verestertes Hydroxy X ist z.B. mit einer starken anorganischen oder organischen Säure verestertes Hydroxy, wie mit einer Mineralsäure, z.B. Halogenwasserstoffsäure, wie Chlorwasserstoff-, Bromwasserstoff- oder Iodwasserstoffsäure, ferner Schwefelsäure, oder Halogenschwefelsäure, z.B. Fluorschwefelsäure, oder mit einer starken organischen Sulfonsäure, wie einer gegebenenfalls, z.B. durch Halogen, wie Fluor, substituierten Niederalkansulfonsäure, z.B. Methylsulfonsäure, oder einer aromatischen Sulfonsäure, z.B. einer gegebenenfalls durch Niederalkyl, wie Methyl, Halogen, wie Brom, und/oder Nitro substituierten Benzolsulfonsäure, z.B. einer Methansulfon-, Trifluormethansulfon- oder p-Toluolsulfonsäure, verestertes Hydroxy, bevorzugterweise ein Chlorid, Bromid oder Iodid.

Die Reaktion wird vorzugsweise in einem inerten organischen Lösungsmittel, z.B. Dimethoxyethan, bei einer Temperatur zwischen -30°C und +100°C, insbesondere -10°C und +70°C, z.B. 0°C und +40°C, erforderlichenfalls in Gegenwart eines säurebindenden Mittels durchgeführt.

Die Abspaltung der Hydroxyschutzgruppe wird durchgeführt, wie bei Verfahren b) beschrieben ist.

Die Ausgangsprodukte der Formel VI und die analogen Verbindungen, worin $R^{20}$ Wasserstoff bedeutet, d.h. die Zwischenprodukte der Formel V,

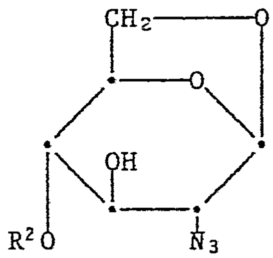

(V)

worin $R^2$ Wasserstoff oder eine Hydroxyschutzgruppe bedeutet, werden z.B. hergestellt, indem man D-Mannose mit Toluolsulfochlorid/Pyridin in 6-O-Tosyl-D-mannose überführt, die erhaltene 6-O-Tosyl-D-mannose mit Natriumethylat in 1,6-Anhydro-β-D-mannopyranose umwandelt, die erhaltene 1,6-Anhydro-β-D-mannopyranose mit Toluolsulfochlorid umsetzt und die erhaltene 1,6-Anhydro-2-O-tosyl-β-D-mannopyranose mit einem geeigneten Azid, z.B. Lithiumazid oder Tetrabutylammoniumazid, umsetzt und, wenn erwünscht, die 4-Hydroxy-Gruppe der erhaltenen 1,6-Anhydro-2-azido-2-desoxy-β-D-glucopyranose mit einer Hydroxyschutzgruppe schützt. Wenn erwünscht, kann die genannte Hydroxyschutzgruppe bereits auf einer der genannten früheren Stufen des Gesamtverfahrens eingeführt werden.

Bei der genannten Herstellung der 1,6-Anhydro-2-azido-2-desoxy-β-D-glucopyranose handelt es sich um ein Methodikverfahren, dass zum Gegenstand der Erfindung gehört.

Verfahren b)

Ein reaktionsfähiges Carbonsäurederivat einer Verbindung der Formel VIII, ist in erster Linie ein reaktionsfähiger Ester, ein reaktionsfähiges Anhydrid oder ein reaktionsfähiges cyclisches Amid, wobei die Aktivierung auch in situ erfolgen kann.

Aktivierte Ester von Säuren sind insbesondere am Verknüpfungskohlenstoffatom des veresternden Restes ungesättigte Ester, z.B. vom Vinylester-Typ, wie eigentliche Vinylester (die man z.B. durch Umesterung eines entsprechenden Esters mit Vinylacetat erhalten kann; Methode des aktivierten Vinylesters), Carbamoylvinylester (die man z.B. durch Behandeln der entsprechenden Säure mit einem Isoxazoliumreagens erhalten kann; 1,2-Oxazolium- oder Woodward-Methode), oder 1-Niederalkoxyvinylester (die man z.B. durch Behandeln der entsprechenden Säure mit einem Niederalkoxyacetylen erhalten kann; Ethoxyacetylen-Methode), oder Ester vom Amidinotyp, wie N,N'-disubstituierte Amidinoester (die man z.B. durch Behandeln der entsprechenden Säure mit einem geeigneten N,N'-disubstituierten Carbodiimid, z.B. N,N'-Dicyclohexylcarbodiimid, erhalten kann; Carbodiimid-Methode), oder N,N-disubstituierte Amidinoester (die man z.B. durch Behandeln der entsprechenden Säure mit einem N,N-disubstituierten Cyanamid erhalten kann; Cyanamid-Methode), geeignete Arylester, insbesondere durch Elektronen-anziehende Substituenten geeignet substituierte Phenylester (die man z.B. durch Behandeln der entsprechenden Säure mit einem geeignet substituierten Phenol, z.B. 4-Nitrophenol, 4-Methylsulfonyl-phenol, 2,4,5-Trichlorphenol, 2,3,4,5,6-Pentachlor-phenol oder 4-Phenyldiazophenol, in Gegenwart eines Kondensationsmittels, wie N,N'-Dicyclohexyl-carbodiimid, erhalten kann; Methode der aktivierten Arylester), Cyanmethylester (die man z.B. durch Behandeln der entsprechenden Säure mit Chloracetonitril in Gegenwart einer Base erhalten kann; Cyanmethylester-Methode), Thioester, insbesondere, gegebenenfalls, z.B. duch Nitro, substituierte Phenyl-thioester (die man z.B. durch Behandeln der entsprechenden Säure mit gegebenenfalls, z.B. durch Nitro, substituierten Thiophenolen, u.a. mit Hilfe der Anhydrid-oder Carbodiimid-Method, erhalten kann; Methode der aktivierten Thiolester), Amino-oder Amidoester (die man z.B. durch Behandeln der entsprechenden Säure mit einer N-Hydroxy-amino- bzw. N-Hydroxy-amido-Verbindung, z.B. N-Hydroxy-succinimid, N-Hydroxy-piperidin, N-Hydroxyphthalimid oder 1-Hydroxy-benztriazol, z.B. nach der Anhydrid- oder Carbodiimid-Methode, erhalten kann; Methode der aktivierten N-Hydroxyester) oder Silylester (die man z.B. durch Behandeln der entsprechenden Säure mit einem Silylierungsmittel, z.B. Hexamethyldisilazan, erhalten kann und die leicht mit Hydroxy-, nicht aber mit Aminogruppen reagieren).

Anhydride von Säuren können symmetrische oder vorzugsweise gemischte Anhydride dieser Säuren sein, so z.B. Anhydride mit anorganischen Säuren, wie Säurehalogenide, insbesondere Säurechloride (die man z.B. durch Behandeln der entsprechenden Säure mit Thionylchlorid, Phosphorpentachlorid oder Oxalylchlorid erhalten kann; Säurechloridmethode), Azide (die man z.B. aus einem entsprechenden Säureester über das entsprechende Hydrazid und dessen Behandlung mit salpetriger Säure erhalten kann; Azidmethode), Anhydride mit Kohlensäurehalbderivaten, wie mit entsprechenden Estern, z.B. Kohlensäureniederalkylhalbestern (die man z.B. durch Behandeln der entsprechenden Säure mit Halogen-, wie Chlorameisensäure-niederalkylestern oder mit einem 1-Niederalkoxycarbonyl-2-niederalkoxy-1,2-dihydro-chinolin, z.B. 1-Niederalkoxycarbonyl-2-ethoxy-1,2-dihydrochinolin, erhalten kann; Methode der gemischten O-Alkyl-kohlensäureanhydride), oder Anhydride mit dihalogenierter, insbesondere dichlorierter Phosphorsäure (die man z.B. durch Behandeln der entsprechenden Säure mit Phosphoroxychlorid erhalten kann; Phosphoroxychloridmethode), oder Anhydride mit organischen Säuren, wie gemischte Anhydride mit organischen Carbonsäuren (die man z.B. durch Behandeln der entsprechenden Säure mit einem gegebenenfalls substituierten Niederalkan- oder

Phenyl-niederalkancarbonsäurehalogenid, z.B. Phenylessigsäure-, Pivalinsäure- oder Trifluoressigsäurechlorid erhalten kann; Methode der gemischten Carbonsäureanhydride) oder mit organischen Sulfonsäuren (die man z.B. durch Behandeln eines Salzes, wie eines Alkalimetallsalzes, der entsprechenden Säure, mit einem geeigneten organischen Sulfonsäurehalogenid, wie Niederalkan- oder Aryl-, z.B. Methan- oder p-Toluolsulfonsäurechlorid, erhalten kann; Methode der gemischten Sulfonsäureanhydride), sowie symmetrische Anhydride (die man z.B. durch Kondensation der entsprechenden Säure in Gegenwart eines Carbodiimids oder von 1-Diethylaminopropin erhalten kann; Methode der symmetrischen Anhydride).

Geeignete cyclische Amide sind insbesondere Amide mit fünfgliedrigen Diazacyclen aromatischen Charakters, wie Amide mit Imidazolen, z.B. Imidazol (die man z.B. durch Behandeln der entsprechenden Säure mit N,N'-Carbonyldiimidazol erhalten kann; Imidazolid-Methode), oder Pyrazolen, z.B. 3,5-Dimethyl-pyrazol (die man z.B. über das Säurehydrazid durch Behandeln mit Acetylaceton erhalten kann; Pyrazolid-Methode).

Wie erwähnt kann ein reaktionsfähiges Carbonsäurederivat einer Verbindung der Formel VIII auch in situ gebildet werden. So kann man z.B. N,N'-disubstituierte Amidinoester in situ bilden, indem man das Gemisch des Ausgangsmaterials der Formel IX und der als Acylierungsmittel verwendeten Säure der Formel VIII in Gegenwart eines geeigneten N,N'-disubstituierten Carbodiimids, z.B. N,N'-Dicyclohexylcarbodiimid, zur Reaktion bringt. Ferner kann man einen Amino-oder Amidoester der als Acylierungsmittel verwendeten Säure in Gegenwart des zu acylierenden Ausgangsmaterials der Formel VIII bilden, indem man das Gemisch der entsprechenden Säure- und Amino-Ausgangsstoffe in Gegenwart eines N,N'-disubstituierten Carbodiimids, z.B. N,N'-Dicyclohexyl-carbodimid, und eines N-Hydroxyamins oder N-Hydroxy-amids, z.B. N-Hydroxysuccinimid oder 1-Hydroxybenztriazol, gegebenenfalls in Anwesenheit einer geeigneten Base, z.B. 4-Dimethylaminopyridin, umsetzt.

In einem reaktionsfähigen Derivat einer Verbindung der Formel IX ist die Aminogruppe z.B. durch Umsetzung mit einem Phosphit, z.B. Diethylchlorphosphit, 1,1-Phenylen-chlorphosphit, Ethyl-dichlorphosphit, Ethylen-chlor-phosphit oder Tetraethylpyrophosphit, oder durch Bindung an Halogencarbonyl, z.B. Chlorcarbonyl, oder in Form einer Isocyanatgruppe aktiviert. Vorzugsweise wird die Reaktion so durchgeführt, dass man ein reaktionsfähiges Carbonsäurederivat einer Verbindung der Formel VIII mit einer Verbindung der Formel IX umsetzt, worin die an der Reaktion teilnehmende Amino- oder Hydroxygruppe in freier Form vorliegt.

In den Verbindungen der Formeln VIII oder IX gegebenenfalls vorhandene funktionelle Gruppen, die vorzugsweise durch leicht abspaltbare Schutzgruppen geschützt werden, sind insbesondere Amino und Guanidino im Rest $R^8$ sowie Carboxy als Rest -CO-$R^7$ und -CO-$R^9$, daneben aber auch Hydroxy im Rest $R^6$ und $R^8$ sowie Hydroxy $OR^2$.

Als Hydroxyschutzgruppen können z.B. die obengenannten verwendet werden. Carboxylgruppen sind üblicherweise in veresterter Form geschützt, wobei solche Estergruppierungen unter schonenden Bedingungen leicht spaltbar sind. In dieser Art geschützte Carboxylgruppen enthalten als veresternde Gruppen in erster Linie in 1-Stellung verzweigte oder in 1- oder 2-Stellung geeignet substituierte Niederalkylgruppen. Bevorzugte, in veresterter Form vorliegende Carboxylgruppen sind u.a. tert.-Niederalkoxycarbonyl, z.B. tert.-Butyloxycarbonyl, Arylmethoxycarbonyl mit einem oder zwei Arylresten, wobei diese gegebenenfalls z.B. durch Niederalkyl, wie tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellen, wie gegebenenfalls, z.B. wie oben erwähnt, substituiertes Benzyloxycarbonyl, z.B. 4-Methoxybenzyloxycarbonyl, oder 4-Nitrobenzyloxycarbonyl, oder gegebenenfalls, z.B. wie oben erwähnt, substituiertes Diphenylmethoxycarbonyl, z.B. Diphenylmethoxycarbonyl oder Di-(4-methoxyphenyl)-methoxycarbonyl, 1-Niederalkoxyniederalkoxycarbonyl, wie Methoxymethoxycarbonyl, 1-Methoxyethoxycarbonyl oder 1-Ethoxymethoxycarbonyl, 1-Niederalkylthioniederalkoxycarbonyl, wie 1-Methylthiomethoxycarbonyl oder 1-Ethylthioethoxycarbonyl, Aroylmethoxycarbonyl, worin die Aroylgruppe gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichlorethoxycarbonyl, 2-Bromethoxycarbonyl oder 2-Iodethoxycarbonyl, oder 2-(trisubstituiertes Silyl)-ethoxycarbonyl, worin die Substituenten unabhängig voneinander je einen gegebenenfalls substituierten, z.B. durch Niederalkyl, Niederalkoxy, Aryl, Halogen, und/oder Nitro substituierten, aliphatischen, araliphatischen, cycloaliphatischen oder aromatischen Kohlenwasserstoffrest, wie entsprechendes, gegebenenfalls substituiertes Niederalkyl, Phenylniederalkyl, Cycloalkyl oder Phenyl, bedeuten, z.B. 2-Triniederalkylsilylethoxycarbonyl, 2-Trimethylsilylethoxycarbonyl oder 2-(Di-n-butyl-methyl-silyl)-ethoxycarbonyl, oder 2-Triarylsilylethoxycarbonyl, wie 2-Triphenylsilylethoxycarbonyl.

Die oben und im folgenden erwähnten organischen Silyl- oder Stannylreste enthalten vorzugsweise Niederalkyl, insbesondere Methyl, als Substituenten der Silizium- oder Zinn-Atome. Entsprechende Silyl-oder Stannylgruppen sind in erster Linie Triniederalkylsilyl, insbesondere Trimethylsilyl, ferner Dimethyl-tert.-butylsilyl, oder entsprechend substituiertes Stannyl, z.B. Tri-n-butylstannyl.

Bevorzugte geschützte Carboxylgruppen sind tert.-Niederalkoxycarbonyl, wie tert.-Butoxycarbonyl, und in erster Linie gegebenenfalls, z.B. wie oben erwähnt, substituiertes Benzyloxycarbonyl, wie 4-Nitrobenzyloxycarbonyl, oder Diphenylmethoxycarbonyl, vor allem 2-(Trimethylsilyl)-ethoxycarbonyl.

Eine geschützte Aminogruppe kann z.B. in Form einer leicht spaltbaren Acylamino-, Arylmethylamino-, verätherten Mercaptoamino-, 2-Acyl-niederalk-1-en-yl-amino-, Silyl- oder Stannylaminogruppe oder als Azidogruppe vorliegen.

In einer entsprechenden Acylaminogruppe Acylaminogruppe ist Acyl beispielsweise der Acylrest einer

organischen Carbonsäure mit z.B. bis zu 18 Kohlenstoffatomen, insbesondere einer gegebenenfalls,z.B. durch Halogen oder Aryl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl oder Propionyl, Halogenniederalkanoyl, wie 2-Halogenacetyl, insbesondere 2-Chlor-, 2-Brom-, 2-Iod-, 2,2,2-Trifluor- oder 2,2,2-Trichloracetyl, gegebenenfalls, z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, insbesondere tert.-Niederalkoxycarbonyl, z.B. tert.-Butyloxycarbonyl, Arylmethoxycarbonyl mit einem oder zwei Arylresten, die vorzugsweise gegebenenfalls, z.B. durch Niederalkyl, insbesondere tert.-Niederalkyl, wie tert.Butyl, Niederalkoxy, wie Methoxy, Hydroxy, Halogen, z.B. Chlor, und/oder Nitro, mono- oder polysubstituiertes Phenyl darstellen, wie gegebenenfalls substituiertes Benzyloxycarbonyl, z.B. 4-Nitro-benzyloxycarbonyl, oder substituiertes Diphenylmethoxycarbonyl, z.B. Benzhydryloxycarbonyl oder Di(4-methoxyphenyl)-methoxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichlorethoxycarbonyl, 2-Bromethoxycarbonyl oder 2-Iodethoxycarbonyl, oder 2-(trisubstituiertes Silyl)-ethoxycarbonyl, worin die Substituenten unabhängig voneinander je einen gegebenenfalls substituierten, z.B. durch Niederalkyl, Niederalkoxy, Aryl, Halogen oder Nitro substituierten, aliphatischen, araliphatischen, cycloaliphatischen oder aromatischen Kohlenwasserstoffrest mit bis zu 15 C-Atomen, wie entsprechendes, gegebenenfalls substituiertes Niederalkyl, Phenylniederalkyl, Cycloalkyl oder Phenyl, bedeuten, z.B. 2-Triniederalkylsilylethoxycarbonyl, wie 2-Trimethylsilylethoxycarbonyl oder 2-(Di-n-butyl-methyl-silyl)-ethoxycarbonyl, oder 2-Triarylsilylethoxycarbonyl, wie 2-Triphenylsilylethoxycarbonyl.

Weitere, als Aminoschutzgruppen in Frage kommende Acylreste sind auch entsprechende Reste organischer Phosphor-, Phosphon- oder Phosphinsäuren, wie Diniederalkylphosphoryl, z.B. Dimethylphosphoryl, Diethylphosphoryl, Di-n-propylphosphoryl oder Diisopropylphosphoryl, Dicycloalkylphosphoryl, z.B. Dicyclohexylphosphoryl, gegebenenfalls substituiertes Diphenylphosphoryl, z.B. Diphenylphosphoryl, gegebenenfalls, z.B. durch Nitro substituiertes Di-(phenylniederalkyl)-phosphoryl, z.B. Dibenzylphosphoryl oder Di-(4-nitrobenzyl)-phosphoryl, gegebenenfalls substituiertes Phenyloxy-phenyl-phosphonyl, z.B. Phenyloxyphenyl-phosphonyl, Diniederalkylphosphinyl, z.B. Diethylphosphinyl, oder gegebenenfalls substituiertes Diphenylphosphinyl, z.B. Diphenylphosphinyl.

In einer Arylmethylaminogruppe, die eine Mono-, Di- oder insbesondere Triarylmethylaminogruppe darstellt, sind die Arylreste insbesondere gegebenenfalls substituierte Phenylreste. Solche Gruppen sind beispielsweise Benzyl-, Diphenylmethyl- und insbesondere Tritylamino.

Eine verätherte Mercaptogruppe in einer mit einem solchen Rest geschützten Aminogruppe ist in erster Linie Arylthio oder Arylniederalkylthio, worin Aryl insbesondere gegebenenfalls, z.B. durch Niederalkyl, wie Methyl oder tert.-Butyl, Niederalkoxy, wie Methoxy, Halogen, wie Chlor, und/oder Nitro substituiertes Phenyl ist. Eine entsprechende Aminoschutzgruppe ist z.B. 4-Nitrophenylthio.

In einem als Aminoschutzgruppe verwendbaren 2-Acyl-niederalk-1-en-1-yl-rest ist Acyl z.B. der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters. Entsprechende Schutzgruppen sind in erster Linie 1-Niederalkanoyl-prop-1-en-2-yl, z.B. 1-Acetyl-prop-1-en-2-yl, oder 1-Niederalkoxycarbonyl-prop-1-en-2-yl, z.B. 1-Ethoxycarbonyl-prop-1-en-2-yl.

Eine Aminogruppe kann auch in protonierter Form geschützt werden; als entsprechende Anionen kommen in erster Linie diejenigen von starken anorganischen Säuren, wie von Halogenwasserstoffsäuren, z.B. das Chlor- oder Bromanion, oder von organischen Sulfonsäuren, wie p-Toluolsulfonsäure, in Frage.

Bevorzugte Aminoschutzgruppen sind Acylreste von Kohlensäurehalbestern, insbesondere tert.-Butyloxycarbonyl, gegebenenfalls, z.B. wie angegeben, substituiertes Benzyloxycarbonyl, z.B. 4-Nitro-benzyloxycarbonyl, oder Diphenylmethoxycarbonyl, oder 2-Halogen-niederalkoxycarbonyl, wie 2,2,2-Trichlorethoxycarbonyl, ferner Trityl oder Formyl.

Guanidinogruppen sind z.B. in Form des Säureadditionssalzes, insbesondere als Hydrochlorid oder als Toluolsulfonat, geschützt.

Die Reaktion kann in an sich bekannter Weise durchgeführt werden, wobei die Reaktionsbedingungen in erster Linie davon abhängen, ob und wie die Carboxylgruppe des Acylierungsmittels aktiviert ist, üblicherweise in Gegenwart eines geeigneten Lösungs- oder Verdünnungsmittels, z.B. Dimethylformamid oder Methylenchlorid, oder eines Gemisches von solchen, und, falls notwendig, in Gegenwart eines Kondensationsmittels, das z.B., wenn die an der Reaktion teilnehmende Carboxylgruppe als Anhydrid vorliegt, auch ein Säure-bindendes Mittel sein kann, unter Kühlen oder Erwärmen. z.B. in einem Temperaturbereich von etwa -30° C bis etwa +100° C, insbesondere von etwa 0° C bis +70° C, vorzugsweise von Raumtemperatur (ca. +20° C) bis +50° C, in einem geschlossenen Reaktionsgefäss und/oder in der Atmosphäre eines Inertgases, z.B. Stickstoff. Uebliche Kondensationsmittel sind z.B. Carbodiimide, beispielsweise N,N'-Diethyl-, N,N'-Dipropyl-, N,N'-Di-cylohexyl- oder N-Ethyl-N'(3-dimethylaminopropyl)-carbodiimid, geeignete Carbonylverbindungen, beispielsweise Carbonyldiimidazol, oder 1,2-Oxazoliumverbindungen, z.B. 2-Ethyl-5-phenyl-1,2-oxazolium3'-sulfonat und 2-tert.-Butyl-5-methyl-isoxazolium-perchlorat, oder eine geeignete Acylaminoverbindung, z.B. 2-Ethoxy-1-ethoxycarbonyl-1,2-dihydrochinolin. Uebliche säurebindende Kondensationsmittel sind z.B. Alkalimetallcarbonate oder -hydrogencarbonate, z.B. Natrium-oder Kaliumcarbonat oder -hydrogencarbonat (üblicherweise zusammen mit einem Sulfat), oder organische Basen, wie üblicherweise sterisch gehinderte Triniederalkylamine, z.B. N,N-Diisopropyl-N-ethyl-amin.

Die Abspaltung der Schutzgruppen, z.B. der Carboxyl-, Amino- oder Hydroxyschutzgruppen, die nicht Bestandteil des gewünschten Endprodukts der Formel I sind, erfolgt in an sich bekannter Weise, z.B. mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig, wobei auch enzymatische Methoden verwendet werden können.

So kann man tert.-Niederalkoxycarbonyl oder in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B. durch Behandeln mit einer geeigneten Säure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, freigesetzt werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zink, oder Metallsalz, wie einem Chrom-II-salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlor-mandelsäure oder Weinsäure, oder eines Alkohols oder Thiols, wobei man vorzugsweise Wasser zugibt, in freies Carboxyl überführen. Durch Behandeln mit einem reduzierenden Metall oder Metallsalz, wie oben beschrieben, kann man auch 2-Halogen-niederalkoxycarbonyl (gegebenenfalls nach Umwandlung einer 2-Brom-niederalkoxycarbonylgruppe in eine entsprechende 2-Iod-niederalkoxycarbonylgruppe) oder Aroylmethoxycarbonyl in freies Carboxyl umwandeln, wobei Aroylmethoxycarbonyl ebenfalls durch Behandeln mit einem nucleophilen, vorzugsweise salzbildenden Reagens, wie Natriumthiophenolat oder Natriumiodid, gespalten werden kann. Substituiertes 2-Silylethoxycarbonyl kann auch durch Behandeln mit einem, das Fluoridanion liefernden Salz der Fluorwaserstoffsäure, wie einem Alkalimetallfluorid, z.B. Natrium-oder Kaliumfluorid, in Anwesenheit eines macrocyclischen Polyethers ("Kronenether"), oder mit einem Fluorid einer organischen quaternären Base, wie Tetra-niederalkylammoniumfluorid oder Triniederalkyl-aryl-ammo niumfluorid, z.B. Tetraethylammoniumfluorid oder Tetrabutylammoniumfluorid, in Gegenwart eines aprotischen polaren Lösungsmittels, wie Dimethylsulfoxid oder N,N-Dimethylacetamid, in freies Carboxyl übergeführt werden.

Verestertes Carboxyl kann auch enzymatisch gespalten werden, z.B. verestertes Lysin, z.B. Lysinmethylester, mittels Trypsin.

Eine geschützte Aminogruppe setzt man in an sich bekannter und je nach Art der Schutzgruppen in verschiedenartiger Weise, vorzugsweise mittels Solvolyse oder Reduktion, frei. 2-Halogen-niederalkoxycarbonylamino (gegebenenfals nach Umwandlung einer 2-Brom-niederalkoxycarbonylaminogruppe in eine 2-Iod-niederalkoxycarbonylaminogruppe), Aroylmethoxycarbonylamino oder 4-Nitrobenzyloxycarbonylamino kann z.B. durch Behandeln mit einem geeigneten chemischen Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagens, wie Natriumthiophenolat, und 4-Nitro-benzyloxycarbonylamino auch durch Behandeln mit einem Alkalimetall-, z.B. Natriumdithionit, gespalten werden. Gegebenenfalls substituiertes Diphenylmethoxycarbonylamino, tert.-Niederalkoxycarbonylamino oder 2-trisubstituiertes Silylethoxycarbonylamino kann durch Behandeln mit einer geeigneten Säure, z.B. Ameisenoder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino oder Formylamino z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, gespalten werden, und eine mit einer organischen Silylgruppe geschützte Aminogruppe kann z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat, des Thioharnstoffs und anschliessende Solvolyse, wie Alkoholyse oder Hydrolyse, des entstandenen Kondensationsprodukts freigesetzt werden. Eine durch 2-substituiertes Silylethoxycarbonyl geschützte Aminogruppe kann auch durch Behandeln mit einem Fluoridanionen liefernden Salz der Fluorwaserstoffsäure, wie oben im Zusammenhang mit der Freisetzung einer entsprechend geschützten Carboxylgruppe angegeben, in die freie Aminogruppe übergeführt werden.

In Form einer Azidogruppe geschütztes Amino wird z.B. durch Reduktion in freies Amino übergeführt, beispielsweise durch katalytische Hydrierung mit Wasserstoff in Gegenwart eines Hydrierkatalyators, wie Platinoxid, Palladium oder Raney-Nickel, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20°C bis 25°C, oder auch unter Kühlen oder Erwärmen, durchgeführt.

Eine durch eine geeignete Acylgruppe, eine organische Silylgruppe oder durch gegebenenfalls substituiertes 1-Phenylniederalkyl geschützte Hydroxygruppe wird analog einer entsprechend geschutzten Aminogruppe freigesetzt. Durch gegebenenfalls substituiertes 1-Phenylniederalkyl, z.B. Benzyl, geschütztes Hydroxy wird vorzugsweise durch katalytische Hydrierung, z.B. in Gegenwart eine Palladium-auf-Kohle-Katalysators freigesetzt. Eine durch 2,2-Dichloracetyl geschützte Hydroxy- bzw. Mercaptogruppe wird z.B. durch basische Hydrolyse, eine durch tert.-Niederalkyl oder durch einen 2-oxa- oder 2-thia-aliphatischen oder -cycloaliphatischen Kohlenwasserstoffrest veretherte Hydroxy- bzw. Mercaptogruppe durch Acidolyse, z.B. durch Behandeln mit einer Mineralsäure oder einer starken Carbonsäure, z.B. Trifluoressigsäure, freigesetzt. Zwei Hydroxygruppen, die zusammen mittels einer vorzugsweise substituierten Methylengruppe, wie durch Niederalkyliden, z.B. Isopropyliden, Cycloalkyliden, z.B. Cyclohexyliden, oder Benzyliden, geschützt sind, können durch saure Solvolyse, besonders in Gegenwart einer Mineralsäure oder einer starken organischen Säure, z.B. Trifluoressigsäure, freigesetzt werden. Mit einem organischen Silylrest, z.B. Trimethylsilyl, verethertes Hydroxy kann auch mit einem Fluoridanionen liefernden Salz der Fluorwasserstoffsäure, z.B. Tetrabutylammoniumfluorid, freigesetzt werden.

In Form des Säureadditionssalzes geschütztes Guanidino kann prinzipiell mit Hilfe einer geeigneten Base freigesetzt werden, wird aber zweckmässigerweise in Form des Säureadditionssalzes belassen.

Bevorzugt werden beim Vorhandensein von mehreren geschützten funktionellen Gruppen die Schutzgruppen so gewählt, dass gleichzeitig mehr als eine solche Gruppe abgespalten werden kann, beispielsweise acidolytisch, wie durch Behandeln mit Trifluoressigsäure oder Ameisensäure, oder reduktiv, wie durch Behandeln mit Zink und Essigsäure, oder mit Wasserstoff und einem Hydrierkatalysator, wie einem Palladium-Kohle-Katalysator. Wenn die gewünschten Endstoffe Schutzgruppen enthalten, z.B. wenn $R^7$ oder $R^9$ Arylmethoxy, wie Benzyloxy, bedeutet, werden diejenigen Schutzgruppen, die nach erfolgter Reaktion abgespalten werden sollen, so gewählt, dass sie regioselektiv wieder abgespalten werden können, z.B. kann mit einem organischen Silylrest verethertes Hydroxy im Rest $R^6$ mit Fluorid freigesetzt werden, wobei eine Arylmethoxy-Schutzgruppe im Rest $R^7$ oder $R^9$ erhalten bleibt.

Verfahren c)

Ein geeignetes Reduktionsmittels ist z.B. Triphenylphosphin in Tetrahydrofuran-Wasser, Schwefelwasserstoff in Pyridin oder Wasserstoff in Gegenwart eines geeigneten Hydrierkatalysators, z.B. Palladium auf Kohlenstoff.

Ein Schutz funktioneller Gruppen kann in der Regel bei Wahl eines entsprechenden Reduktionsmittels vermieden werden.

Die Reduktion wird vorzugsweise in einem Temperaturbereich von etwa 0°C bis etwa +70°C, z.B. bei Raumtemperatur (ca. 20°C), durchgeführt.

Zusatzoperationen: Salze von Verbindungen der Formel I mit mindestens einer salzbildenden Gruppe können in an sich bekannter Weise hergestellt werden. So kann man Salze von Verbindungen der Formel I mit sauren Gruppen durch Umsetzung mit einer geeigneten Base, z.B. durch Behandeln mit geeigneten Metallverbindungen, wie Alkalimetallsalzen von geeigneten organischen Carbonsäuren, z.B. dem Natriumsalz der α-Ethyl-capronsäure, oder mit geeigneten anorganischen Alkali- oder Erdalkalimetallsalzen, insbesondere solchen, die sich von einer schwachen und vorzugsweise flüchtigen Säure ableiten, z.B. Natriumhydrogencarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauschreagens. Innere Salze von Verbindungen der Formel I, welche z.B. eine freie Carboxylgruppe und eine freie Aminogruppe enthalten, können z.B. durch Neutralisieren von Salzen, wie Säureadditionssalzen, auf den isoelektrischen Punkt, z.B. mit schwachen Basen, oder durch Behandeln mit flüssigen Ionenaustauschern gebildet werden.

Salze können in üblicher Weise in die freien Verbindungen übergeführt werden, Metall- und Ammoniumsalze z.B. durch Behandeln mit geeigneten Säuren, und Säureadditionssalze z.B. durch Behandeln mit einem geeigneten basischen Mittel.

Gemische von Isomeren können in an sich bekannter Weise, z.B. durch fraktionierte Kristallisation, Chromatographie etc. in die einzelnen Isomeren aufgetrennt werden.

Die Veresterung von freiem Hydroxy im Rest $R^6$ und/oder $R^8$ und die Acylierung von freiem Amino $R^8$ erfolgt in an sich bekannter Weise durch Umsetzung mit einer Niederalkancarbonsäure oder vorzugsweise einem reaktionsfähigem Derivat davon erforderlichenfalls unter intermediärem Schutz anderer funktioneller Gruppen.

Zur Veresterung oder Amidierung von freiem Carboxy $-COR^7$ und/oder $-COR^9$ kann eine Verbindung der Formel I, worin $R^7$ und/oder $R^9$ Hydroxy bedeuten, wobei in dieser Verbindung vorhandene freie funktionelle Gruppen mit Ausnahme der an der Reaktion teilnehmenden Gruppe(n) erforderlichenfalls durch leicht abspaltbare Schutzgruppen geschützt sind, oder ein reaktionsfähiges Säurederivat davon verestert oder amidiert werden. Dabei kann die Veresterung oder Amidierung so durchgeführt werden, dass man ein reaktionsfähiges Säurederivat mit dem entsprechenden Alkohol oder Ammoniak umsetzt. Vorzugsweise wird aber die Veresterung so durchgeführt, dass man eine Verbindung der Formel I, worin $R^7$ und/oder $R^9$ Hydroxy bedeuten, mit einem reaktionsfähigen Veresterungsmittel umsetzt.

Geeignete Mittel zur Veresterung sind beispielsweise entsprechende Diazoverbindungen, wie gegebenenfalls substituierte Diazoniederalkane, z.B. Diazomethan, Diazoethan oder Diazo-n-butan. Diese Reagenzien

werden in Gegenwart eines geeigneten inerten Lösungsmittels, wie eines aliphatischen, cycloaliphatischen oder aromatischen Kohlenwasserstoffs, wie Hexan, Cyclohexan, Benzol, oder Toluol, eines halogenierten aliphatischen Kohlenwasserstoffs, z.B. Methylenchlorid, oder eines Ethers, wie eines Diniederalkylethers, z.B. Diethylether, oder eines cyclischen Ethers, z.B. Tetrahydrofuran oder Dioxan, oder eines Lösungsmittelgemisches, und, je nach Diazoreagens, unter Kühlen, bei Raumtemperatur oder unter leichtem Erwärmen, ferner, wenn notwendig, in einem geschlossenen Gefäss und/oder unter einer Inertgas-, z.B. Stickstoffatmosphäre, zur Anwendung gebracht.

Weitere geeignete Mittel zur Veresterung sind Ester entsprechender Alkohole, in erster Linie solche mit starken anorganischen oder organischen Säuren, wie Mineralsäuren, z.B. Halogenwasserstoffsäuren, wie Chlorwasserstoff-, Bromwasserstoff- oder lodwasserstoffsäure, ferner Schwefelsäure, oder Halogen-schwefelsäure, z.B. Fluorschwefelsäure, oder starken organischen Sulfonsäuren, wie gegebenenfalls z.B. durch Halogen, wie Fluor, substituierten Niederalkansulfonsäuren, oder aromatischen Sulfonsäuren, wie z.B. gegebenenfalls durch Niederalkyl, wie Methyl, Halogen, wie Brom, und/oder Nitro substituierten Benzolsulfonsäuren, z.B. Methansulfon-, Trifluormethansulfon- oder p-Toluolsulfonsäure. Solche Ester sind u.a. Niederalkylhalogenide, Diniederalkylsulfate, wie Dimethylsulfat, ferner Fluorsulfonsäureester, wie -niederalkylester, z.B. Fluorsulfonsäuremethylester, oder gegebenenfalls Halogen-substituierte Methansulfonsäure-niederalkylester, z.B. Trifluormethansulfonsäuremethylester. Sie werden üblicherweise in Gegenwart eines inerten Lösungsmittels, wie eines gegebenenfalls halogenierten, wie chlorierten, aliphatischen, cycloaliphatischen oder aromatischen Kohlenwasserstoffs, z.B. Methylenchlorid, eines Ethers, wie Dioxan oder Tetrahydrofuran, oder eines Gemisches davon verwendet. Dabei wendet man vorzugsweise geeignete Kondensationsmittel, wie Alkalimetallcarbonate oder -hydrogencarbonate, z.B. Natrium-oder Kaliumcarbonat oder -hydrogencarbonat (üblicherweise zusammen mit einem Sulfat), oder organische Basen, wie üblicherweise sterisch gehinderte Trinierderalkylamine, z.B. N,N-Diisopropyl-N-ethyl-amin (vorzugsweise zusammen mit Halogensulfonsäure-niederalkylestern oder gegebenenfalls halogensubstituierten Methansulfonsäureniederalkylestern) an, wobei unter Kühlen, bei Raumtemperatur oder unter Erwärmen, z.B. bei Temperaturen von etwa -20° C bis etwa 50° C, und, wenn notwendig, in einem geschlossenen Gefäss und/oder in einer Inertgas-, z.B. Stickstoffatmosphäre, gearbeitet wird.

Weitere Mittel zur Veresterung sind entsprechende trisubstituierte Oxoniumsalze (sogenannte Meerweinsalze), oder disubstituierte Carbenium- oder Haloniumsalze, worin die Substituenten die verethernden Reste sind, beispielsweise Triniederalkyloxoniumsalze, sowie Diniederalkoxycarbenium- oder Diniederalkylhaloniumsalze, insbesondere die entsprechenden Salze mit komplexen, fluorhaltigen Säuren, wie die entsprechenden Tetrafluorborate, Hexafluorphosphate, Hexafluorantimonate, oder Hexachlorantimonate. Solche Reagentien sind z.B. Trimethyloxonium- oder Triethyloxonium-hexafluorantimonat, -hexachlorantimonat, -hexafluorphosphat oder -tetrafluorborat, Dimethoxycarbeniumhexafluorphosphat oder Dimethylbromoniumhexafluorantimonat. Man verwendet diese Mittel vorzugsweise in einem inerten Lösungsmittel, wie einem Ether oder einem halogenierten Kohlenwasserstoff, z.B. Diethylether, Tetrahydrofuran oder Methylenchlorid, oder in einem Gemisch davon, wenn notwendig, in Gegenwart einer Base, wie einer organischen Base, z.B. eines, vorzugsweise sterisch gehinderten. Triniederalkylamins, z.B. N,N-Diisopropyl-N-ethyl-amin, und unter Kühlen, bei Raumtemperatur oder unter leichtem Erwärmen, z.B. bei etwa -20°C bis etwa 50°C, wenn notwendig, in einem geschlossenen Gefäss und/oder in einer Inertgas-, z.B. Stickstoffatmosphäre.

Eine bevorzugte Ausführungsform dieser Veresterungsreaktion ist die Umsetzung eines Cäsiumsalzes einer Verbindung der Formel I, worin $R^7$ und/oder $R^9$ für Hydroxy stehen, mit dem als Veresterungskomponente gewünschten Alkohol, worin die Hydroxylgruppe in reaktionsfähiger veresterter Form vorliegt.

Die Verbindungen der Formel III, worin $R^{10}$ Azido und $R^{13}$ Hydroxy oder Niederalkoxy bedeuten, oder Salze von solchen Verbindungen mit mindestens einer salzbildenden Gruppe werden z.B. hergestellt, indem man eine Verbindung der Formel VI, worin $R^{20}$ durch Phenyl, welches unsubstituiert oder substituiert sein kann, und/oder Niederalkyl trisubstituiertes Silyl bedeutet, oder ein reaktionsfähiges Derivat davon mit einer Verbindung der Formel VII gemäss dem oben beschriebenen Verfahren a) umsetzt, die Verbindung der Formel III aus dem erhaltenen Gemisch abtrennt, und, wenn erwünscht, eine erhaltene Verbindung der Formel III mit mindestens einer salzbildenden Gruppe in ihr Salz überführt oder ein erhaltenes Salz einer Verbindung der Formel III in die freie Verbindung umwandelt.

Aus den so erhältlichen Verbindungen der Formel III, worin $R^{10}$ Azido und $R^{13}$ Hydroxy bedeuten, stellt man die Verbindungen der Formel III, worin $R^{10}$ Azido und $R^{13}$ einen Rest der Formel IV bedeuten, analog Verfahren b) her.

Die Verbindungen der Formel III, worin $R^{10}$ Amin bedeutet, erhält man analog Verfahren c) durch Reduktion der Azidogruppe.

Die oben beschriebenen Verfahren, inklusive die Verfahren zur Abspaltung von Schutzgruppen und die zusätzlichen Verfahrensmassnahmen, werden, wenn nicht anders angegeben, in an sich bekannter Weise, z.B. in Anwesenheit von vorzugsweise inerten Lösungs- und Verdünnungsmitteln, wenn notwendig, in Anwesenheit von Kondensationsmitteln oder Katalysatoren, bei erniedrigter oder erhöhter Temperatur, z.B. in einem Temperaturbereich von etwa -20°C bis etwa +120°C, insbesondere von etwa 0°C bis etwa +70°C, vorzugsweise von etwa +10°C bis etwa +40°C, hauptsächlich bei Raumtemperatur, in einem geschlossenen Gefäss und/oder in einer Inertgas-, z.B. Stickstoffatmosphäre, durchgeführt.

Dabei sind unter Berücksichtigung aller im Molekül befindlichen Substituenten, wenn erforderlich, z.B. bei Anwesenheit leicht hydrolysierbarer Reste, besonders schonende Reaktionsbedingungen, wie kurze

Reaktionszeiten, Verwendung von milden sauren oder basischen Mitteln in niedriger Konzentration, stöchiometrische Mengenverhältnisse, Wahl geeigneter Katalysatoren, Lösungsmittel, Temperatur- und/oder Druckbedingungen, anzuwenden.

Die Erfindung betrifft auch diejenigen Ausführungsformen des Verfahrens, bei denen man von einer auf irgendeiner Stufe des Verfahrens als Zwischenprodukt erhältlichen Verbindung ausgeht und die fehlenden Verfahrensschritte durchführt oder das Verfahren auf irgendeiner Stufe abbricht oder einen Ausgangsstoff in Form eines reaktionsfähigen Derivats oder Salzes verwendet. Dabei geht man vorzugsweise von solchen Ausgangsstoffen aus, die verfahrensgemäss zu den oben als besonders wertvoll beschriebenen Verbindungen führen.

Neue Ausgangsstoffe und/oder Zwischenprodukte sowie Verfahren zu ihrer Herstellung sind ebenfalls Gegenstand der vorliegenden Erfindung. Vorzugsweise werden solche Ausgangsstoffe verwendet und die Reaktionsbedingungen so gewählt, dass man zu den in dieser Anmeldung als besonders bevorzugt aufgeführten Verbindungen gelangt.

Die Erfindung betriffft auch pharmazeutische Präparate, die eine wirksame Menge, insbesondere eine zur Therapie von Tumorerkrankungen wirksame Menge, der Aktivsubstanz zusammen mit pharmazeutisch verwendbaren Trägerstoffen enthalten, die sich zur topischen, z.B. intranasalen, enteralen, z.B. oralen oder rektalen, oder parenteralen Verabreichung eignen, und anorganisch oder organisch, fest oder flüssig sein können. So verwendet man Tabletten oder Gelatinekapseln, welche den Wirkstoff zusammen mit Verdünnungsmitteln, z.B. Lactose, Dextrose, Sukrose, Mannitol, Sorbitol, Cellulose und/oder Glycerin, und/oder Schmiermitteln, z.B. Kieselerde, Talk, Stearinsäure oder Salze davon, wie Magnesium- oder Calciumstearat, und/oder Polyethylenglykol, enthalten. Tabletten können ebenfalls Bindemittel, z.B. Magnesiumaluminiumsilikat, Stärken, wie Mais-, Weizen-oder Reisstärke, Gelatine, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel enthalten. Ferner kann man die pharmakologisch wirksamen Verbindungen der vorliegenden Erfindung in Form von parenteral verabreichbaren Präparaten oder von Infusionslösungen verwenden. Solche Lösungen sind vorzugsweise isotonische wässrige Lösungen oder Suspension, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz-und/oder Emulgiermittel, Löslichkeitsvermittler, Salze zur Regulierung des osmotischen Drucks und/oder Puffer enthalten. Die vorliegenden pharmazeutischen Präparate, die, wenn erwünscht, weitere pharmakologisch wirksame Stoffe, wie Antibiotika, enthalten können, werden in an sich bekannter Weise, z.B. mittels konventioneller Misch-, Granulier-, Dragier-, Lösungs- oder Lyophilisierungsverfahren, hergestellt und enthalten von etwa 0,01 % bis 20 %, insbesondere von etwa 0,1 % bis etwa 10 %, in erster Linie zwischen 0,5 % und 5 %, des bzw. der Aktivstoffe, wobei eine Wirkstoffkonzentration unterhalb von 1 % insbesondere für topisch zu applizierende Präparate geeignet ist.

Als topisch zu applizierende Darreichungsformen sind die folgenden bevorzugt: Crèmes, Salben oder Pasten, z.B. Salben zur intranasalen Applikation oder Lippenstifte, oder vorzugsweise isotonische, sterile und physiologisch verträgliche Lösungen, z.B. Augentropfen, vorzugsweise in Mikrobehältern zur einmaligen Verwendung, oder Sprays zur Anwendung im Mund- und Rachenraum.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei Verarbeitung des Wirkstoffs als Lösung wird dieser in der Regel vor der Emulgierung in einer der beiden Phasen gelöst; bei Verarbeitung als Suspension wird er nach der Emulgierung mit einem Teil der Grundlage vermischt und dann dem Rest der Formulierung beigegeben.

Die nachfolgenden Beispiele illustrieren die Erfindung, ohne sie in irgendeiner Form einzuschränken. Die $R_f$-Werte auf Kieselgeldünnschichtplatten (Firma Merck, Darmstadt, Deutschland) ermittelt. Das Verhältnis der Laufmittel in den verwendeten Laufmittelgemischen zueinander ist in Volumenanteilen (V/V), Temperaturen sind in Grad Celsius angegeben. Die Konzentration, c, der Substanz im Lösungsmittel(gemisch) ist im Falle der optischen Drehung in Prozent (Gewicht/Volumen) angegeben.

<u>Abkürzungen</u>:

abs. = absolut
Boc = tert.Butyloxycarbonyl
Et = Ethyl
EtOH = Ethanol
HV = Hochvakuum
i.Vak.= im Vakuum
Me = Methyl
MeOH = Methanol
proz. = prozentig
PTFE = Polytetrafluorethylen, Teflon®
RT = Raumtemperatur
Smp. = Schmelzpunkt

Zers. = Zersetzung

Beispiel 1: Zu 482 mg (1,13 mMol) 1,6-Anhydro-2-azido-2-desoxy-4-0-(tert.-butyl-diphenyl-silyl)-β-D-glucopyranose in 5 ml Dimethoxyethan gibt man 35 mg 78proz. NaH in Paraffin (Fluka) und rührt 20 Minuten bei 0° unter Feuchtigkeitsausschluss. Dann gibt man weitere 53 mg NaH-Dispersion zu und tropft anschliessend bei 0° unter gutem Rühren eine Lösung von 284 mg 2-Methylsulfonyl-L-milchsäure in 7 ml Dimethoxyethan zu. Nach 1 Stunde bei 0° gibt man weitere 26 mg NaH-Dispersion zu und rührt noch 5 Stunden bei 0°. Dann neutralisiert man mit 0,1 N Salzsäure, dampft im Vakuum ein und chromatographiert an Keiselgel mit Chloroform-Methanol-Wasser (90:10:1). Man erhält 1,6-Anhydro-2-azido-2-desamino-4-O-(tert.-butyl-diphenylsilyl)-D-muraminsäure-natriumsalz, $R_f = 0{,}47$ ($CHCl_3$:MeOH:$H_2O$ = 90:10:1), $[\alpha]_D^{20} = +38{,}5 \pm 1°$ (c = 0,987 ; $CHCl_3$) und 2-[1,6-Anhydro-2-azido-2-desoxy-3-O-(tert.-butyl-diphenyl-silyl)-β-D-glucopyranos-4-yl-oxy]-propionsäure-natriumsalz, $R_f = 0{,}41$ ($CHCl_3$:MeOH:$H_2O$ = 90:10:1), $[\alpha]_D^{20} = +44{,}2 \pm 1{,}6°$ (c = 0,615 ; MeOH). Beide Salze werden durch Ansäuern bis pH = 3, Ausschütteln mit Essigsäureethylester, Waschen der organischen Phase mit Wasser, Trocknen mit $Na_2SO_4$ und Eindampfen im Vakuum in die freien Säuren überführt.

Das Ausgangsmaterial erhält man folgendermassen:

Stufe 1.1: Man löst 270 g (1,5 Mol) D-Mannose in 2 Liter Pyridin bei 20°, kühlt auf -5° und gibt bei dieser Temperatur während 1 Stunde eine Lösung von 395 g (2,08 Mol) Toluolsulfochlorid in 1 Liter Pyridin tropfenweise zu. Man rührt die Lösung anschliessend bei -5° 20 Stunden. Dann bringt man den pH-Wert der Lösung bei 0°-3° mit 4normaler Natronlauge auf 6,9-7,0 (Verbrauch 480 ml 4normaler NaOH). Dann dampft man im Vakuum bei 40° zur Trockne, nimmt den Rückstand in 4 Liter Wasser auf und extrahiert viermal mit je 500 ml Diethylether zur Entfernung des Gemisches der Ditosylate. Die Etherphasen extrahiert man zweimal mit Wasser. Die Wasserphasen werden mit NaCl gesättigt und sechsmal mit je 500 ml 1-Butanol extrahiert, die organische Phase mit $Na_2SO_4$ getrocknet und im Vakuum eingedampft. Eine Probe des Rückstandes, der aus 6-O-Tosyl-D-mannose besteht, wird aus Essigsäureethylester umkristallisiert und zeigt den Smp. 108-109°; $R_f = 0{,}62$ ($CHCl_3$:MeOH:$H_2O$ = 70:30:5).

Stufe 1.2: 590 g 6-O-Tosyl-D-mannose, verunreinigt mit Natriumtosylat (~210 g), löst man in 2 Liter Ethanol und tropft dazu bei -10° unter Argon 1,3 Liter einer einnormalen ethanolischen Natriumethylatlösung. Der pH-Wert der Reaktionslösung, gemessen an einer mit Wasser verdünnten Probe, liegt dabei zwischen 12,5 und 13. Wenn kein Ethylat mehr verbraucht wird, rührt man noch 30 Minuten bei -10°. Dann bringt man den pH-Wert mit 2N Salzsäure bei -10° auf 7,2 und dampft im Vakuum zur Trockne.

Das Gemisch aus 1,6-Anhydro-β-D-mannopyranose und Natriumtosylat wird als 5%ige wässrige Lösung über die 5fache Menge Amberlite IRA-400, Chloridform, gegeben, das Eluat, bestehend aus NaCl und 1,6-Anhydro-β-D-mannopyranose, im Vakuum eingedampft und in $CHCl_3$-MeOH-$H_2O$ (65:30:5) über die 9fache Menge Kieselgel filtriert. Man erhält nach Eindampfen des Eluats im Vakuum 1,6-Anhydro-β-D-mannopyranose als Sirup; $R_f = 0{,}51$ ($CHCl_3$:MeOH:$H_2O$ = 70:30:5).

Stufe 1.3: 140 g eines trockenen Gemisches aus 1,6-Anhydro-β-D-mannopyranose und Natriumtosylat mit einem Gehalt von etwa 0,17 Mol Anhydrozucker, wie es aus der Aufarbeitung von 1,6-Anhydro-β-D-mannopyranose erhalten wird, löst man in einer Mischung aus 500 ml Pyridin und 500 ml Methylenchlorid, saugt von ungelösten Kristallen (Natriumtosylat) ab und versetzt das Filtrat bei -20° innert 60 Minuten mit einer Lösung von 40 g (0,20 Mol) Toluolsulfochlorid in einer Mischung aus 200 ml Methylenchlorid und 200 ml Pyridin. Nach 36 Stunden bei -20° gibt man tropfenweise 100 ml Wasser zu und engt im Vakuum bei maximal 40° Badtemperatur ein. Den sirupösen Rückstand verteilt man zwischen Essigsäureethylester und 2 N Zitronensäure, dann schüttelt man mit Wasser neutral, trocknet die organische Phase mit $Na_2SO_4$ und dampft im Vakuum ein. Das so erhaltene Produkt wird durch Verreiben mit Diethylether von Ditosylat befreit und aus Aceton-Diethylether umkristallisiert, worauf man 1,6-Anhydro-2-O-tosyl-β-D-mannopyranose erhält; Smp. 144-145° (vgl. J. Chem. Soc. 1957, 2271: Smp. 146-147°).

Stufe 1.4: 23 g 1,6-Anhydro-2-O-tosyl-β-D-mannopyranose und 18 g Lithiumazid erhitzt man 24 Stunden auf 120° in 100 ml Dimethylformamid. Nach Eindampfen im Vakuum verteilt man den Rückstand zwischen Essigsäureethylester und Wasser. Nach dem Trocknen der organischen Phase über $Na_2SO_4$ und Eindampfen erhält man ein gelbliches Kristallisat, das aus Essigsäureethylester umkristallisiert wird. Man erhält 1,6-Anhydro-2-azido-2-desoxy-β-D-glucopyranose; Smp. 126-127°, $R_f = 0{,}56$ ($CHCl_3$:Aceton = 1:1), $[\alpha]_D^{20} = -29{,}8 \pm 0{,}9°$ (c = 1,167;EtOH).

Stufe 1.5: 520 mg (2,8 mMol) 1,6-Anhydro-2-azido-2-desoxy-β-D-glucopyranose und 420 mg (6,2 mMol) Imidazol löst man in 7 ml Acetonitril und versetzt mit 7,9 µl (3,08 mMol) tert.-Butyl-diphenyl-chlor-silan bei 0°. Nach 18 Stunden bei 4° dampft man im Vakuum zur Trockne, nimmt den Rückstand mit Essigsäureethylester auf und schüttelt zweimal mit Wasser aus. Nach Trocknen der organischen Phase mit $Na_2SO_4$ und Einengen auf ~2 ml versetzt man mit Petrolether. Die erhaltenen farblosen Kristalle werden aus wenig Diethylether durch Zugabe von Hexan umkristallisiert, worauf man 1,6-Anhydro-2-azido-2-desoxy-4-O-(tert.-butyl-diphenyl-silyl)-β-D-glucopyranose erhält; Smp. 138-139°, $R_f = 0{,}47$ (Hexan:Essigsäureethylester = 7:3), $[\alpha]$ $^{0}$ = -10,4 $\pm 0{,}9°$ (c = 1,107; $CHCl_3$).

Beispiel 2: 520 mg (1 mMol) 1,6-Anhydro-2-azido-2-desamino-4-O-(tert.-butyl-diphenyl-silyl)-β-D-muraminsäure-natriumsalz (s. Beispiel 1) behandelt man in 15 ml Tetrahydrofuran mit 2 g Tetrabutylammoniumfluorid auf Kieselgel (1,16 mMol F-/g) 15 Stunden bei Raumtemperatur, filtriert, wäscht mit 20 ml Tetrahydrofuran nach, engt im Vakuum ein und extrahiert den Rückstand mit Diethylether. Die erhaltene Verbindung, 1,6-Anhydro-2-azido-2-desamino-β-D-muraminsäure-natriumsalz, kann für weitere Umsetzungen so eingesetzt werden. Ansäuern mit verdünnter Salzsäure bis pH = 3 und Extraktion mit Essigsäureethylester führt zur freien Säure; Smp. 191-193°, $R_f$ = 0,18 ($CHCl_3$:MeOH:$H_2O$ = 90:10:1), $[\alpha]_D^{20}$ = + 54,9 ±1,1° (c = 929; MeOH).

Beispiel 3: 280 mg (1 mMol) 1,6-Anhydro-2-azido-2-desamino-β-D-muraminsäure-natriumsalz (s. Beispiel 2), 240 mg (1,1 mMol) N,N'-Di-cyclohexylcarbodiimid und 150 mg (1,1 mMol) N-Hydroxy-benztriazol löst man in 10 ml absolutem Dimethylformamid und versetzt unter Eiskühlung mit 76,5 µl (1,0 mMol) Trifluoressigsäure. Nach 3 Stunden bei Raumtemperatur gibt man zur Reaktionslösung 330 mg (1,1 mMol) L-α-Amino-butyryl-D-glutaminsäure-dimethylester-hydrochlorid und 0,15 ml Triethylamin. Nach 20 Stunden bei Raumtemperatur dampft man im Vakuum ein, nimmt den Rückstand mit Essigsäureethylester auf, saugt von den Kristallen (Dicyclohexylharnstoff) ab, und extrahiert das Filtrat dreimal mit 5prozentiger $NaHCO_3$-Lösung und zweimal mit Wasser. Nach dem Trocknen der organische Phase über $Na_2SO_4$ und Eindampfen kristallisiert man aus Aceton-Diethylether um. Man erhält so 1,6-Anhydro-2-azido-2-desamino-β-D-muramyl-L-α-amino-butyryl-D-glutaminsäure-dimethylester als farblose Kristalle; Smp. 93-96°, $[\alpha]_D^{20}$ = + 0,3° (c = 1; MeOH), $R_f$ = 0,42 (Essigsäureethylester:EtOH = 95:5).

Beispiel 4: 500 mg (1 mMol) 1,6-Anhydro-2-azido-2-desamino-β-D-muramyl-L-α-amino-butyryl-D-glutaminsäure-dimethylester (s. Beispiel 3) in 10 ml Tetrahydrofuran versetzt man mit 350 mg (1,33 mMol) Triphenyl-phosphin und 5 ml zweinormaler $NH_4OH$-Lösung. Nach 15 Stunden bei Raumtemperatur dampft man im Vakuum zur Trockne, nimmt mit Tetrahydrofuran auf und versetzt mit 1 ml einnormaler Salzsäure. Nach dem Eindampfen der Lösung, Extraktion des Rückstandes mit Essigsäureethylester und Umkristallisieren des Rückstandes aus EtOH-Essigsäureethylester erhält man 1,6-Anhydro-β-D-muramyl-L-α-amino-butyryl-D-glutaminsäure-dimethylester-hydrochlorid in Form farbloser Kristalle; $R_f$ = 0,4 (Wasser:Acetonitril = 6:4), Smp. 195-197° (Zers.), $[\alpha]_D^{20}$ = + 13,1 ± 0,9° (c = 1,17; MeOH).

Beispiel 5: Analog Beispiel 1 erhält man aus 1,6-Anhydro-2-azido-2-desoxy-4-O-(tert.-butyl-diphenyl-silyl)-β-D-glucopyranose und Bromessigsäuremethylester 1,6-Anhydro-2-azido-2-desamino-4-O-(tert.-butyl-diphenyl-silyl)-β-desmethylmuraminsäure-methylester, $R_f$ = 0,20 ($CHCl_3$), $[\alpha]_D^{20}$ = + 32,5 ±1,1° (c = 0,924; $CHCl_3$) und 2-[1,6-Anhydro-2-azido-2-desoxy-3-O-(tert.-butyl-diphenyl-silyl)-β-D-glucopyranos-4-yl-oxy]-essigsäure-methylester; $R_f$ = 0,14 ($CHCl_3$), $[\alpha]_D^{20}$ = + 56,8 ± 1,4° (c = 0,706; $CHCl_3$).

Beispiel 6: Analog Beispiel 2 erhält man aus 1,6-Anhydro-2-azido-2-desamino-4-O-(tert.-butyl-diphenyl-silyl)-β-desmethylmuraminsäuremethylester 1,6-Anhydro-2-azido-2-desamino-β-desmethylmuraminsäurenatriumsalz: $R_f$ = 0,2 ($CHCl_3$:MeOH:$H_2O$ = 70:30:3).

Beispiel 7: Analog Beispiel 3 erhält man aus 1,6-Anhydro-2-azido-2-desamino-β-desmethylmuraminsäure-natriumsalz (s. Beispiel 6) 1,6-Anhydro-2-azido-2-desamino-β-desmethylmuramyl-L-α-amino-butyryl-D-glutaminsäure-dimethylester.

Beispiel 8: Aus 1,6-Anhydro-2-azido-2-desamino-β-desmethylmuramyl-L-α-amino-butyryl-D-glutaminsäure-dimethylester erhält man mit Triphenylphosphin und Ansäuren mit verdünnter HCl 1,6-Anhydro-β-desmethylmuramyl-L-α-amino-butyryl-D-glutaminsäure-dimethylester-hydrochlorid.

Beispiel 9: 600 mg 1,6-Anhydro-4-O-trityl-β-desmethylmuramyl-L-alanyl-D-isoglutamin lässt man in einer Mischung aus 16 ml Methylenchlorid und 4 ml Trifluoressigsäure bei Raumtemperatur 1 Stunde stehen, dampft im Vakuum ein, extrahiert den Rückstand dreimal mit Petrolether und chromatographiert ihn über Kieselgel (Typ 60, Merck, 0,04-0,063 mm) mit $CHCl_3$-MeOH-$H_2O$ (70:30:5). Die substanzhaltigen Fraktionen werden gefriergetrocknet, worauf man 1,6-Anhydro-β-desmethylmuramyl-L-alanyl-D-isoglutamin erhält; $R_f$ = 0,11 ($CHCl_3$MeOH:$H_2O$ = 70:30:5), Smp. 81-86°, $[\alpha]_D^{20}$ = - 16,3° ±2° (c = 0,49; $H_2O$).

Stufe 9.1: 55,8 g (0,1 Mol) 1,6-Anhydro-2-O-tosyl-4-O-trityl-β-D-mannopyranose und 500 ml einer einmolaren Lösung von Tetrabutylammoniumazid in Toluol erhitzt man 14 Stunden bei 100° unter Argon. Dann dampft man im Vakuum ein, nimmt den Rückstand in Chloroform auf, kühlt auf 0° und erhält Kristalle von 1,6-Anhydro-2-azido-2-desoxy-O-trityl-β-D-glucopyranose; Smp. 222-223°, $[\alpha]_D^{20}$ = - 37,5° ± 1,1° (c = 0,947; $CHCl_3$) $R_f$ = 0,42 (Hexan:Essigsäureethylester = 7:3).

Stufe 9.2: 30 g (70 mMol) 1,6-Anhydro-2-azido-2-desoxy-4-O-trityl-β-D-glucopyranose erwärmt man in 300 ml abs. Dimethoxyethan mit 2,2 g NaH (75 % in Weissöl) 30 Minuten auf 40°. Nach dem Abkühlen auf Raumtemperatur gibt man weitere 3,4 g NaH in Weissöl und anschliessend 14,4 g (103 mMol) Bromessigsäure in 500 ml Dimethoxyethan so zu, dass die Temperatur 40° nicht übersteigt. Anschliessend rührt man 6 Stunden bei 50°. Das abgekühlte Reaktionsgemisch nutscht man ab, wäscht den Niederschlag mit 50 ml kaltem Dimethoxyethan und erhält so nach dem Trocknen des Niederschlags bei 45° und 13 Pa (0,1 Torr) 1,6-Anhydro-2-azido-2-desamino-4-O-trityl-β-desmethyl muraminsäure-natriumsalz; Smp. 218-220° (Zers.), $[\alpha]_D^{20}$ = + 6,9° ± 1° (c = 0,99; $CHCl_3$:MeOH:$H_2O$ = 70:30:5), $R_f$ = 0,51 ($CHCl_3$:MeOH:$H_2O$ = 80:20:1). Die Substanz wird umkristallisiert durch Lösen in Aceton-$H_2O$ (1:1) bei 40° und Zugabe von 3 Volumenteilen Wasser.

Stufe 9.3: 800 mg (1,6 mMol) 1,6-Anhydro-2-azido-2-desamino-4-O-trityl-β-desmethylmuraminsäure-natriumsalz, suspendiert in einer Mischung aus 6 ml Dimethylformamid und 4 ml Methylenchlorid, versetzt man mit 603 mg (1,76 mMol) L-Alanyl-D-isoglutamin-benzyl-ester-hydrochlorid, 285 mg (1,8 mMol) 1-Hydroxybenztriazol und 500 mg N,N'-Dicyclohexylcarbodiimid in 6 ml Dimethylformamid und 4 ml Methylenchlorid. Man erhält eine klare Lösung, die man nach 17 Stunden bei Raumtemperatur im Vakuum zur Trockne dampft. Den Rückstand extrahiert man bei Raumtemperatur dreimal mit Petrolether und verteilt ihn zwischen Essigsäureethylester und Wasser. Die vereinigten Essigesterphasen trocknet man über $Na_2SO_4$, dampft im Vakuum ein und chromatographiert den erhaltenen Sirup an 15 g Kieselgel (Typ 60, Merck, 0,04-0,063 mm) mit

$CHCl_3$-MeOH (95:5) als Elutionsmittel. Man erhält farblose Kristalle von 1,6-Anhydro-2-azido-2-desamino-4-O-trityl-β-desmethylmuramyl-L-alanyl-D-isoglutamin-benzylester, die man aus Essigsäureethylester umkristallisiert; Smp. 155-156°, $[\alpha]_D^{20}$ = + 36,7° ± 0,9° (c = 1,04; $CHCl_3$), $R_f$ = 0,35 ($CHCl_3$:MeOH = 95:5).

Stufe 9.4: 800 mg 1,6-Anhydro-2-azido-2-desamino-4-O-trityl-β-desmethylmuramyl-L-alanyl-D-isoglutamin-benzylester hydriert man in 80 ml Methanol-$H_2O$ (9:1) mit 0,2 g PdO auf Kohlenstoff bei Raumtemperatur. Nach 3 Stunden filtriert man den Katalysator ab, dampft das Filtrat ein und verreibt den Rückstand mit Diethylether, worauf man 1,6-Anhydro-4-O-trityl-β-desmethylmuramyl-L-alanyl-D-isoglutamin als farbloses Pulver erhält; Smp. 173-179°, $[\alpha]_D^{20}$= + 2,9° ± 1° (c = 0,99; $CHCl_3$:MeOH:$H_2O$ = 70:30:5), $R_f$ = 0,55 ($CHCl_3$:MeOH:$H_2O$ = 70:30:5).

Beispiel 10: 20 mg N-Acetyl-1,6-anhydro-4-O-trityl-β-desmethylmuramyl-L-alanyl-D-isoglutamin-benzylester erwärmt man 15 Stunden auf 60° in 1 ml Dimethoxyethan-Wasser-Trifluormethansulfonsäure (70:30:3). Man erhält N-Acetyl-desmethylmuramyl-L-alanyl-D-glutaminsäure; $R_f$ = 0,14 ($CHCl_3$:Methanol:Wasser = 70:30:5).

**Patentansprüche**

1. Ein 1,6-Anhydro-β-D-glucopyranose-derivat der Formel I,

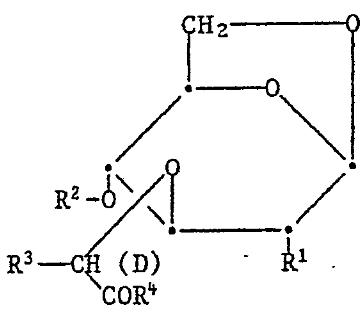

(I)

worin $R^1$ Azido oder Amino bedeutet, $R^2$ Wasserstoff oder eine Hydroxyschutzgruppe bedeutet, $R^3$ Wasserstoff oder Niederalkyl bedeutet und $R^4$ Hydroxy, Niederalkoxy oder einen Rest der Formel II bedeutet,

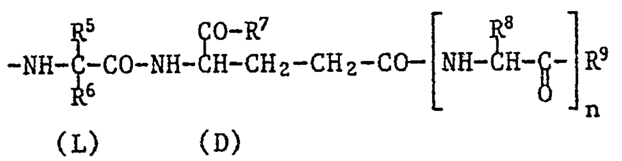

(II)

worin n für 0 oder 1 steht, $R^5$ Wasserstoff oder Niederalkyl bedeutet, $R^6$ Wasserstoff oder Niederalkyl, das unsubstituiert oder durch Hydroxy, Niederalkanoyloxy oder Phenyl substituiert ist, bedeutet, $R^7$ und $R^9$ unabhängig voneinander Hydroxy, Amino, Niederalkoxy, Arylniederalkoxy oder Niederalkanoyloxyniederalkoxy bedeuten und $R^8$ Wasserstoff oder unsubstituiertes oder durch Amino, Hydroxy, Niederalkanoylamino, Niederalkanoyloxy oder Guanidino substituiertes Niederalkyl bedeutet, oder ein Salz einer solchen Verbindung mit mindestens einer salzbildenden Gruppe.

2. Eine Verbindung der Formel I nach Anspruch 1, worin $R^1$ Azido oder Amino, $R^2$ Wasserstoff oder eine Hydroxyschutzgruppe, $R^3$ Wasserstoff oder Niederalkyl und $R^4$ Hydroxy oder Niederalkoxy bedeuten, oder ein Salz einer solchen Verbindung mit mindestens einer salzbildenden Gruppe.

3. Eine Verbindung der Formel I nach Anspruch 1 oder 2, worin $R^1$ Azido bedeutet, oder ein Salz einer solchen Verbindung mit mindestens einer salzbildenden Gruppe.

4. Eine Verbindung der Formel I nach Anspruch 1 oder 2, worin $R^1$ Amino bedeutet, oder ein Salz einer solchen Verbindung mit mindestens einer salzbildenden Gruppe.

5. Eine Verbindung der Formel I nach einem der Ansprüche 1-4, worin $R^3$ Wasserstoff bedeutet, oder ein Salz einer solchen Verbindung mit mindestens einer salzbildenden Gruppe.

6. Eine Verbindung der Formel I nach einem der Ansprüche 1-5, worin $R^2$ eine über ein gesättigtes C-Atom gebundene Hydroxyschutzgruppe bedeutet, oder ein Salz einer solchen Verbindung mit mindestens einer salzbildenden Gruppe.

7. Eine Verbindung der Formel I nach Anspruch 6, worin $R^2$ Allyl, Benzyl oder Tetrahydropyranyl

bedeutet, oder ein Salz einer solchen Verbindung mit mindestens einer salzbildenden Gruppe.

8. Eine Verbindung der Formel I nach Anspruch 6, worin $R^2$ Trityl bedeutet, oder ein Salz einer solchen Verbindung mit mindestens einer salzbildenden Gruppe.

9. Eine Verbindung der Formel I nach einem der Ansprüche 1-8, worin $R^4$ Hydroxy bedeutet, oder ein Salz davon.

10. Eine Verbindung der Formel I nach Anspruch 1, worin $R^1$ Amino, $R^2$ Wasserstoff, $R^3$ Wasserstoff oder Niederalkyl und $R^4$ einen Rest der Formel II bedeuten, oder ein Salz einer solchen Verbindung mit mindestens einer salzbildenden Gruppe.

11. Eine Verbindung der Formel I nach Anspruch 1 oder 10, worin $R^4$ einen Rest der Formel II bedeutet, worin n für 0 oder 1 steht, $R^5$ Wasserstoff oder $C_{1-3}$-Alkyl bedeutet, $R^6$ Wasserstoff, $C_{1-4}$-Alkyl oder durch Hydroxy, Niederalkanoyloxy oder Phenyl substituiertes $C_{1-2}$-Alkyl bedeutet, $R^7$ und $R^9$ unabhängig voneinander Hydroxy, Amino, Niederalkoxy, Niederalkanoyloxymethoxy, oder durch 1-3 Phenylreste substituiertes Methoxy bedeuten und $R^8$ Wasserstoff, $C_{1-4}$-Alkyl, 4-Amino-n-butyl, 4-Niederalkanoylamino-n-butyl, 3-Guanidino-n-propyl oder durch Hydroxy oder Niederalkanoyloxy substituiertes $C_{1-2}$-Alkyl bedeutet, oder ein Salz einer solchen Verbindung mit mindestens einer salzbildenden Gruppe.

12. Eine Verbindung der Formel I nach Anspruch 10 oder 11, worin $R^4$ einen Rest der Formel II bedeutet, worin n für 0 steht, $R^5$ Wasserstoff bedeutet, $R^6$ $C_{1-4}$-Alkyl bedeutet und $R^7$ und $R^9$ unabhängig voneinander Hydroxy, Amino, $C_{1-4}$-Alkoxy oder Benzyloxy bedeuten, oder ein pharmazeutisch verwendbares Salz einer solchen Verbindung mit mindestens einer salzbildenden Gruppe.

13. 1,6-Anhydro-2-azido-2-desamino-4-O-trityl-β-desmethylmuraminsäure oder ein Salz davon nach Anspruch 1.

14. 1,6-Anhydro-2-azido-2-desamino-β-desmethylmuraminsäure oder ein Salz davon nach Anspruch 1.

15. 1,6-Anhydro-2-azido-2-desamino-β-D-muraminsäure oder ein Salz davon nach Anspruch 1.

16. Eine Verbindung der Formel I,

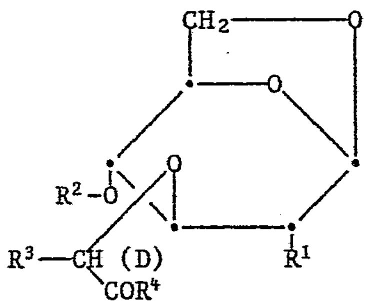

(I)

worin $R^1$ Amino, $R^2$ Wasserstoff, $R^3$ Wasserstoff oder Niederalkyl und $R^4$ einen Rest der Formel II bedeuten,

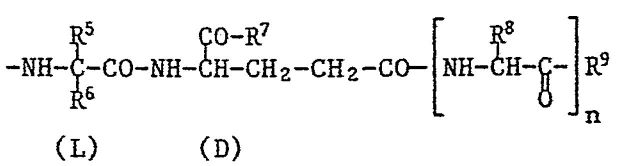

(II)

worin n für 0 oder 1 steht, $R^5$ Wasserstoff oder Niederalkyl bedeutet, $R^6$ Wasserstoff oder Niederalkyl, das unsubstituiert oder durch Hydroxy, Niederalkanoyloxy oder Phenyl substituiert ist, bedeutet, $R^7$ und $R^9$ unabhängig voneinander Hydroxy, Amino, Niederalkoxy, Arylniederalkoxy oder Niederalkanoyloxyniederalkoxy bedeuten und $R^8$ Wasserstoff oder unsubstituiertes oder durch Amino, Hydroxy, Niederalkanoylamino, Niederalkanoyloxy oder Guanidino substituiertes Niederalkyl bedeutet, oder ein pharmazeutisch verwendbares Salz einer solchen Verbindung mit mindestens einer salzbildenden Gruppe zur Anwendung in einem Verfahren zur therapeutischen Behandlung von Warmblütern.

17. Pharmazeutisches Präparat, das eine Verbindung der Formel I,

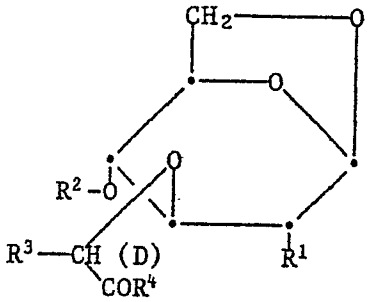

(I)

worin $R^1$ Amino, $R^2$ Wasserstoff, $R^3$ Wasserstoff oder Niederalkyl und $R^4$ einen Rest der Formel II bedeuten,

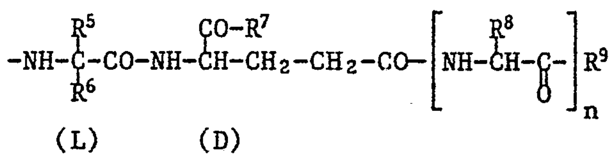

(II)

worin n für 0 oder 1 steht, $R^5$ Wasserstoff oder Niederalkyl bedeutet, $R^6$ Wasserstoff oder Niederalkyl, das unsubstituiert oder durch Hydroxy, Niederalkanoyloxy oder Phenyl substituiert ist, bedeutet, $R^7$ und $R^9$ unabhängig voneinander Hydroxy, Amino, Niederalkoxy, Arylniederalkoxy oder Niederalkanoyloxyniederalkoxy bedeuten und $R^8$ Wasserstoff oder unsubstituiertes oder durch Amino, Hydroxy, Niederalkanoylamino, Niederalkanoyloxy oder Guanidino substituiertes Niederalkyl bedeutet, oder ein pharmazeutisch verwendbares Salz einer solchen Verbindung mit mindestens einer salzbildender Gruppe zusammen mit pharmazeutischem Trägermaterial enthält.

18. Verwendung einer Verbindung der Formel I,

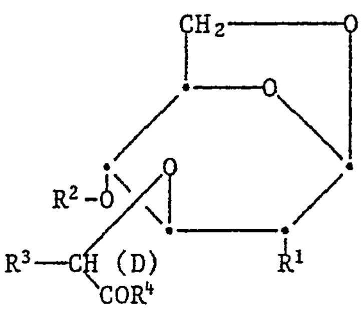

(I)

worin $R^1$ Amino, $R^2$ Wasserstoff, $R^3$ Wasserstoff oder Niederalkyl und $R^4$ einen Rest der Formel II bedeuten,

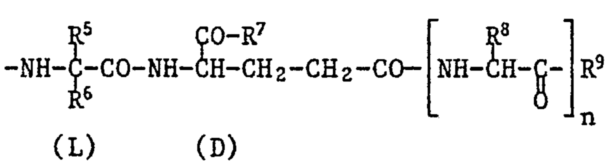

(II)

worin n für 0 oder 1 steht, $R^5$ Wasserstoff oder Niederalkyl bedeutet, $R^6$ Wasserstoff oder Niederalkyl, das unsubstituiert oder durch Hydroxy, Niederalkanoyloxy oder Phenyl substituiert ist, bedeutet, $R^7$ und $R^9$ unabhängig voneinander Hydroxy, Amino, Niederalkoxy, Arylniederalkoxy oder Niederalkanoyloxynieder-

alkoxy bedeuten und $R^8$ Wasserstoff oder unsubstituiertes oder durch Amino, Hydroxy, Niederalkanoylamino, Niederalkanoyloxy oder Guanidino substituiertes Niederalkyl bedeutet, oder eines pharmazeutisch verwendbaren Salzes einer solchen Verbindung mit mindestens einer salzbildenden Gruppe zur Herstellung von pharmazeutischen Präparaten, die zur Stimulierung des Immunsystems von Warmblütern bestimmt sind.

19. Verfahren zur Herstellung einer Verbindung der Formel I,

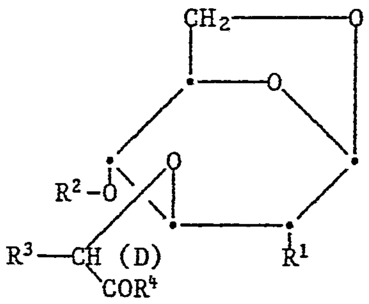

(I)

worin $R^1$ Azido oder Amino bedeutet, $R^2$ Wasserstoff oder eine Hydroxyschutzgruppe bedeutet, $R^3$ Wasserstoff oder Niederalkyl bedeutet und $R^4$ Hydroxy, Niederalkoxy oder einen Rest der Formel II bedeutet,

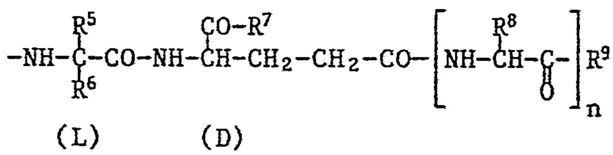

(II)

worin n für 0 oder 1 steht, $R^5$ Wasserstoff oder Niederalkyl bedeutet, $R^6$ Wasserstoff oder Niederalkyl, das unsubstituiert oder durch Hydroxy, Niederalkanoyloxy oder Phenyl substituiert ist, bedeutet, $R^7$ und $R^9$ unabhängig voneinander Hydroxy, Amino, Niederalkoxy, Arylniederalkoxy oder Niederalkanoyloxyniederalkoxy bedeuten und $R^8$ Wasserstoff oder unsubstituiertes oder durch Amino, Hydroxy, Niederalkanoylamino, Niederalkanoyloxy oder Guanidino substituiertes Niederalkyl bedeutet, oder eines Salzes einer solchen Verbindung mit mindestens einer salzbildenden Gruppe, dadurch gekennzeichnet, dass man

a) zur Herstellung einer Verbindung der Formel I, worin $R^1$ Azido und $R^4$ Hydroxy oder Niederalkoxy bedeuten, eine Verbindung der Formel VI,

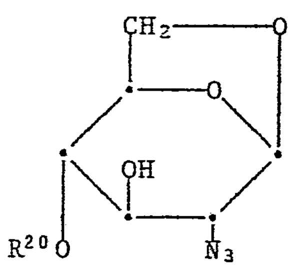

(VI)

worin $R^{20}$ eine Hydroxyschutzgruppe bedeutet, oder ein reaktionsfähiges Derivat davon mit einer Verbindung der Formel VII,

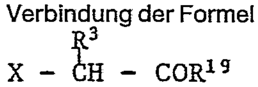

(VII)

worin X eine reaktionsfähige veresterte Hydroxygruppe bedeutet, $R^{19}$ Hydroxy oder Niederalkoxy bedeutet und $R^3$ eine der obengenannten Bedeutungen hat, umsetzt und, wenn erwünscht, zur Herstellung von Verbindungen der Formel I, worin $R^2$ Wasserstoff bedeutet, die Hydroxyschutzgruppe $R^{20}$ abspaltet, oder

b) zur Herstellung einer Verbindung der Formel I, worin $R^1$ Azido und $R^4$ einen Rest der Formel II bedeuten, eine Verbindung der Formel VIII,

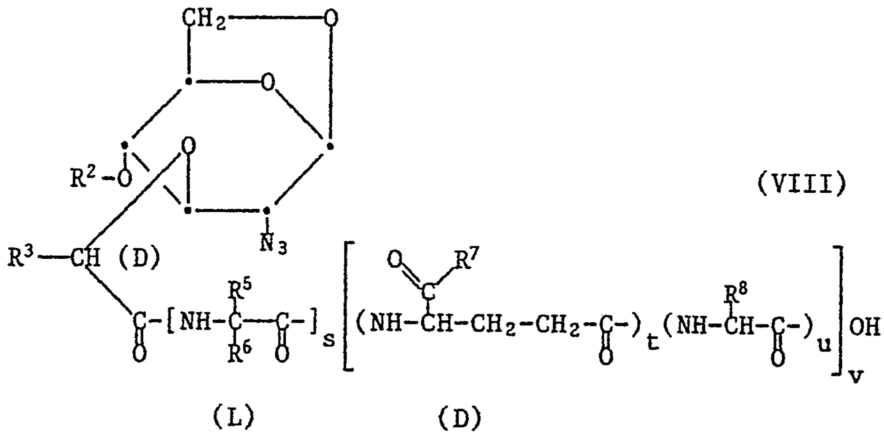

(VIII)

worin s, t, u und v unabhängig voneinander für 0 oder 1 stehen und worin die Substituenten die obengenannten Bedeutungen haben, wobei in einer Verbindung der Formel VIII vorhandene freie funktionelle Gruppen mit Ausnahme der Gruppe, die an der Reaktion teilnehmen soll, erforderlichenfalls durch leicht abspaltbare Schutzgruppen geschützt sind, oder ein reaktionsfähiges Carbonsäurederivat davon mit einer Verbindung der Formel IX,

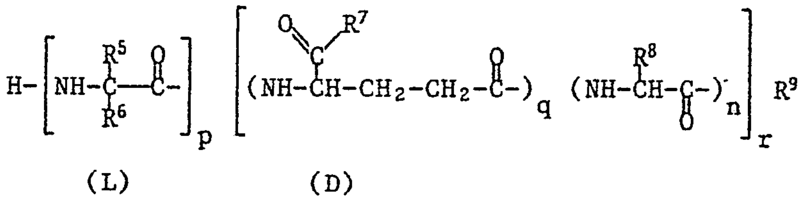

(IX)

worin p, q und r unabhängig voneinander für 0 oder 1 stehen und die übrigen Symbole und Substituenten die obengenannten Bedeutungen haben, wobei in einer Verbindung der Formel IX vorhandene freie funktionelle Gruppen mit Ausnahme der Gruppe, die an der Reaktion teilnehmen soll, erforderlichenfalls durch leicht abspaltbare Schutzgruppen geschützt sind, wobei p, q und r für 1 stehen, wenn im Reaktionspartner der Formel VIII s und v für 0 stehen, oder p für 0 und q und r für 1 stehen, wenn s für 1 und v für 0 stehen, oder p und q für 0 und r für 1 stehen, wenn s, t und v für 1 und u für 0 stehen oder zur Herstellng von Verbindungen der Formel I, worin n für 1 steht, p und r für 0 stehen, wenn s, t, u und v für 1 stehen, oder mit einem reaktionsfähigen Derivat davon umsetzt und vorhandene Schutzgruppen erforderlichenfalls abspaltet, oder

c) zur Herstellung einer Verbindung der Formel I, worin $R^1$ Amino bedeutet, eine Verbindung der Formel I, worin $R^1$ Azido bedeutet und die übrigen Substituenten die obengenannten Bedeutungen haben, wobei vorhandene frei funktionelle Gruppen mit Ausnahme der Gruppe, die an der Reaktion teilnehmen soll, erforderlichenfalls durch leicht abspaltbare Schutzgruppen geschützt sind, mit einem geeigneten Reduktionsmittel in die Aminoverbindung überführt und vorhandene Schutzgruppen erforderlichenfalls abspaltet,

und, wenn erwünscht, nach Durchführung eines der Verfahren a-c) eine erhaltene Verbindung der Formel I mit mindestens einer salzbildenden Gruppe in ihr Salz überführt oder ein erhaltenes Salz einer Verbindung der Formel I in die freie Verbindung umwandelt, und/oder ein erhaltenes Isomerengemisch auftrennt, und/oder zwecks Ueberführung einer erhaltenen Verbindung der Formel I in eine andere Verbindung der Formel I freies Hydroxy im Rest $R^6$ und/oder $R^8$ verestert oder freies Amino im Rest $R^8$ acyliert oder freies Carboxy $-COR^7$ und/oder $-COR^9$ verestert oder amidiert.